(19) Europäisches Patentamt
European Patent Office
Office européen des brevets

(11) EP 4 106 720 B1

(12) # FASCICULE DE BREVET EUROPEEN

(45) Date de publication et mention de la délivrance du brevet:
**27.12.2023 Bulletin 2023/52**

(21) Numéro de dépôt: **21704807.3**

(22) Date de dépôt: **15.02.2021**

(51) Classification Internationale des Brevets (IPC):
***A61K 8/9789*** *(2017.01)* ***A61K 36/28*** *(2006.01)*
***A61P 17/00*** *(2006.01)* ***A61Q 19/00*** *(2006.01)*
***A61P 17/16*** *(2006.01)* ***A61Q 19/08*** *(2006.01)*
***A61Q 5/00*** *(2006.01)*

(52) Classification Coopérative des Brevets (CPC):
**A61K 8/9789; A61K 36/28; A61P 17/16; A61Q 5/00; A61Q 19/00; A61Q 19/002; A61Q 19/007; A61Q 19/08**

(86) Numéro de dépôt international:
**PCT/EP2021/053646**

(87) Numéro de publication internationale:
**WO 2021/165204 (26.08.2021 Gazette 2021/34)**

(54) **LYSAT DE CELLULES DEDIFFERENCIEES DE LA PLANTE HELICHRYSUM STOECHAS ADMINISTRABLE PAR VOIE TOPIQUE POUR HYDRATER LA PEAU**

TOPISCH VERABREICHBARES LYSAT VON DEDIFFERENZIERTEN ZELLEN DER PFLANZE HELICHRYSUM STOECHAS ZUR FEUCHTIGKEITSVERSORGUNG DER HAUT

TOPICALLY ADMINISTRABLE LYSATE OF DEDIFFERENTIATED CELLS OF THE PLANT HELICHRYSUM STOECHAS FOR MOISTURIZING SKIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.02.2020 FR 2001547**

(43) Date de publication de la demande:
**28.12.2022 Bulletin 2022/52**

(73) Titulaire: **Société d'Exploitation de Produits pour les Industries Chimiques SEPPIC**
**75321 Paris cedex 07 (FR)**

(72) Inventeurs:
- **LE GELEBART, Erwan**
  **22260 QUEMPER-GUÉZENNEC (FR)**
- **BIZE, Cécile**
  **81100 CASTRES (FR)**

(74) Mandataire: **Air Liquide**
**L'Air Liquide S.A.**
**Direction de la Propriété Intellectuelle**
**75, Quai d'Orsay**
**75321 Paris Cedex 07 (FR)**

(56) Documents cités:
**FR-A1- 3 040 625**

- **DATABASE GNPD [Online] MINTEL; 31 octobre 2018 (2018-10-31), anonymous: "Cloudberry Kit", XP055718965, extrait de www.gnpd.com Database accession no. 6090571**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 4 106 720 B1

## Description

La présente invention est relative à un lysat de cellules dédifférenciées de la plante Helichrysum stoechas administrable par voie topique pour hydrater la peau humaine et/ou le cuir chevelu et/ou les lèvres, plus particulièrement les peaux sèches, réactives et/ou sensibles.

L'hydratation est une propriété de la peau faisant intervenir des mécanismes complexes qui ne sont pas encore tous connus à ce jour. Ces mécanismes sont localisés dans toutes les strates de la peau.

La peau constitue l'interface entre le milieu intérieur humain (ou organisme humain) et l'environnement extérieur. De ce fait, et avec la flore qui la recouvre et l'habite, la peau a notamment pour fonction d'assurer une mission de protection de l'organisme humain, en formant une véritable barrière qui est vitale. En particulier la peau permet de lutter contre la déshydratation en limitant la diffusion de l'eau hors de l'organisme.

En raison de sa position d'interface avec le milieu extérieur, la peau est soumise à des sollicitations quotidiennes nombreuses, telles que par exemple le contact avec des vêtements, les changements de températures, l'exposition aux rayonnements ultra-violets de la lumière du soleil, les changements de degrés d'hygrométrie, le contact avec certains produits chimiques irritants, le contact avec des produits chimiques considérés comme des agents polluants.

La peau est composée de couches de différents tissus :

- L'épiderme, composé de kératinocytes, est sa partie la plus externe, puis vient
- Le derme, qui est un tissu conjonctif composé principalement de fibroblastes et de la matrice extracellulaire, et
- L'hypoderme, constitué d'adipocytes, qui est la partie la plus profonde et la plus éloignée du milieu extérieur.

La peau assure diverses fonctions dans l'intérêt de l'ensemble du système qu'elle abrite parmi lesquelles on peut retenir :

- Une fonction de barrière mécanique pour garantir l'intégrité du milieu intérieur de l'organisme,
- Une fonction émonctorielle visant à sécréter de la sueur à base d'eau, de sels et de déchets acides,
- Une fonction de régulation de la température du corps, et contient bien d'autres mécanismes de régulation, comme par exemple son mécanisme d'adaptation et de protection face aux rayonnements ultra-violets (coloration pigmentaire adaptative par la production de la mélanine), comme par exemple un système de veille immunitaire par la présence de macrophages, de cellules dendritiques.

La peau humaine constitue aussi la première image offerte au regard d'autrui.

Par conséquent, l'amélioration de son aspect est un sujet de préoccupation constant pour les êtres humains. La peau est le reflet d'un état de bien-être, souvent associé à la jeunesse, et a contrario d'un état de fatigue et/ou de vieillissement. Il en résulte que la préservation, l'amélioration de l'état de la couche la plus extérieure de la peau, à savoir l'épiderme, constitue un centre d'intérêt majeur pour les recherches menées par les industries cosmétiques.

A la périphérie de l'épiderme, se trouve une couche cornée supérieure, nommée le *stratum corneum,* qui est la première couche de l'épiderme à subir les stress d'origine externe, comme les variations de conditions climatiques extérieures (température, hygrométrie, rayonnements ultra-violet, agents polluants) ou les sollicitations mécaniques.

C'est pourquoi, l'amélioration de l'apparence ou le maintien de la bonne apparence de la peau humaine, consiste notamment à maintenir un état d'hydratation du *stratum corneum* à un niveau optimal et satisfaisant. Cela permet en plus d'éviter les inconvénients esthétiques et physiologiques liés à un état de sécheresse de la peau. D'autre part, la peau sèche ou très sèche est un état chronique qui peut être lié à un dysfonctionnement d'ordre génétique : il s'agit d'un type de peau, comme il existe la peau de type grasse, la peau de type mixte...

Mais au-delà de la sécheresse constitutionnelle, il existe aussi des états de sécheresse cutanée déclenchés par des facteurs externes, climatiques, environnementaux, causés par des pathologies cutanées (eczéma atopique, psoriasis...), des maladies générales (thyroïde, diabète, carence nutritionnelles) et/ou des traitements médicaux. La peau ne remplit plus efficacement sa fonction de barrière cutanée : elle se déshydrate. Ainsi, la sécheresse cutanée est due à un affaiblissement, une altération de la fonction barrière de la peau.
Les peaux sèches sont principalement caractérisées par :

- Une desquamation légère ;
- la présence de rugosités ;
- la présence de rougeurs ;
- l'apparition de gerçures ou de micro-craquelures ;
- Une perte d'élasticité ;
- Le développement d'une sensibilisation de la peau, qui se manifeste par de légers tiraillements, des picotements,

des démangeaisons, des douleurs.

Par conséquent, assurer une bonne hydratation des couches superficielles de l'épiderme, du *stratum corneum* permet :

- De maintenir l'élasticité de la peau ;
- D'assurer la protection de la peau contre les dommages extérieurs ;
- Un bon fonctionnement des enzymes hydrolytiques intervenants dans le mécanisme de desquamation de façon à assurer un renouvellement optimal de l'épiderme ;
- De contribuer à la fonction de barrière de la peau de façon optimale.

Les peaux sensibles se définissent par une réactivité particulière de la peau.

Cette réactivité cutanée se traduit classiquement par la manifestation de signes d'inconfort, comme les rougeurs, en réponse à la mise en contact du sujet avec un élément déclenchant qui peut avoir diverses origines.

Elle a été définie il y a peu comme suit: "un syndrome défini par l'apparition de sensations désagréables (sensations de picotement, brûlure, douleur, prurit et de fourmillement) en réponse à des stimuli qui normalement ne devraient pas provoquer de telles sensations. Ces sensations ne peuvent pas être expliquées par des lésions liées à des maladies de peau. La peau peut sembler normale ou présenter en plus de l'érythème. La peau sensible peut affecter toutes les régions du corps, et en particulier le visage ».

Des facteurs environnementaux tels que l'exposition à des rayons ultraviolets ou infrarouges, et/ou à la pollution atmosphérique , et/ou à des variations brutales de température et/ou au vent, ou le mode de vie (habitudes alimentaires ou application de produits cosmétiques en surface de la peau), ou encore des facteurs physiologiques comme le stress, les hormones endogènes, ont été reconnus comme pouvant induire ou aggraver les symptômes des peaux sensibles. Deux raisons principales peuvent expliquer les symptômes des peaux sensibles: d'une part une augmentation de la perméabilité du *stratum corneum,* d'autre part une sécrétion excessive de certains neuromédiateurs par les terminaisons des nerfs superficiels et des cytokines pro-inflammatoires.

Au sens de la présente invention, les peaux sensibles couvrent les peaux irritables et les peaux intolérantes.

Une peau intolérante est une peau qui réagit par des sensations d'échauffement, de tiraillements, de fourmillements et/ou de rougeurs, à différents facteurs tels que l'application de produits cosmétiques ou dermatologiques ou de savon. En général, ces signes sont associés à un érythème et à une peau hyper-séborrhéique ou acnéique, voire même rosacéiforme, avec ou sans dartres.

Une peau irritable est une peau qui réagit par un prurit, c'est-à-dire par des démangeaisons ou par des picotements, à différents facteurs tels que l'environnement, les émotions, les aliments, le vent, les frottements, le rasoir, l'eau dure à forte concentration de calcaire, les variations de température, l'humidité ou la laine.

Partant de là, un problème qui se pose est de fournir un composé permettant d'hydrater la peau et/ou le cuir chevelu.

Une solution selon la présente invention est un lysat (Ly) de cellules dédifférenciées de la plante *Helichrysum stoechas* administrable par voie topique pour améliorer l'hydratation de la peau et/ou du cuir chevelu et/ou des lèvres.

Par « administrable par voie topique » on entend que le lysat est formulé pour être administrable par voie topique.

Le lysat (Ly) de cellules dédifférenciées de la plante Helichrysum stoechas sera le principe actif permettant l'amélioration de l'hydratation de la peau et/ou du cuir chevelu et/ou des lèvres.

Au sens de la présente invention, une amélioration de l'hydratation de la peau, et plus particulièrement du *stratum corneum,* peut se concevoir par mise en contact de la peau avec des composés qui possèdent un « effet hydratant ».

Par « effet hydratant », on entend au sens de la présente invention :

- Une réduction de la perte insensible en eau, et/ou
- Une diminution des espaces intercellulaires entre les kératinocytes de la couche basale de l'épiderme, et/ou
- Une augmentation de l'épaisseur du *Stratum corneum,* et/ou
- La restauration ou l'amélioration de la fonction barrière, et/ou
- L'augmentation des zones lacunaires, et/ou
- une augmentation du taux d'hydratation du *stratum corneum,*

résultant de l'application topique d'une substance chimique ou d'une composition chimique sur la surface de la peau à traiter.

Selon un aspect particulier, la présente invention concerne un lysat (Ly) de cellules dédifférenciées de la plante *Helichrysum stoechas* administrable par voie topique pour prévenir ou ralentir l'apparition des signes extérieurs du vieillissement de la peau humaine, comme par exemple l'apparition des rides, des ridules, d'une altération du microrelief,

du manque d'élasticité et/ou de tonus, du manque de densité et/ou de fermeté de la peau humaine.

En effet, il y a un lien entre vieillissement de la peau et hydratation. L'eau est un composant essentiel de la peau et du cuir chevelu. Si l'épiderme est bien hydraté, gorgé d'eau, il montrera un aspect repulpé et lisse. A l'inverse, la déshydratation a des effet inverse sur sa qualité, notamment par l'apparition de rides, de poches sous les yeux, d'un teint gris. La déshydratation est une des conséquences naturelles du vieillissement. L'épiderme a la faculté de capter l'humidité, et de conserver une hydratation optimale. Or cette capacité décroît avec l'âge. Le vieillissement des cellules cutanées provoque donc une diminution de la proportion d'acide hyaluronique, dont le rôle est de « piéger » dans l'épiderme les molécules d'eau. La baisse de sécrétion hormonale qui intervient autour de la cinquantaine, s'ajoute à cela. Ainsi, la peau perd progressivement son niveau d'hydratation.

Par ailleurs, le lysat selon l'invention pourra être administrable par voie topique pour hydrater la peau après le rasage du visage ou pour hydrater le cuir chevelu après un nettoyage et/ou traitement du cuir chevelu.

De préférence, le lysat (Ly) est issu de l'homogénéisation haute pression de la culture de cellules dédifférenciées de la plante Helichrysum stoechas.

Le procédé d'obtention du lysat de cellules dédifférenciées de la plante Helichrysum stoechas selon l'invention comprendra de préférence les étapes suivantes :

a) Préparation de l'échantillon stérile de plante Helichrysum stoechas,
b) Callogenèse
c) Mise en suspension
d) Sélection d'une suspension fine
e) Production de biomasse
f) Homogénéisation Haute Pression de la culture
g) Stabilisation du Lysat de culture de cellules dédifférenciées

A partir de l'étape e) les étapes a) b) c) d) n'ont plus besoin d'être reconduites car elle mène à la culture de cellules dédifférenciées utilisables à l'infini pour produire de la biomasse dans la mesure où les besoins physiologiques des cellules sont respectés.

Les étapes b) c) d) et e) doivent être réalisées en conditions stériles afin de maintenir la culture dans des conditions axéniques.

Les étapes sont détaillées comme suit :

a) Préparation de l'échantillon *d'Helichrysum stoechas* stérile

Prélever quelques feuilles d'Helichrysum stoechas, et découper ces feuilles en fragments de quelques millimètres. Stériliser les fragments de feuilles par immersion dans des bains successifs contenant :

- Un mélange d'éthanol 70% contenant un détergent tel que du Tween 80 afin d'améliorer l'efficacité de la stérilisation pendant une durée de 5 minutes, puis dans
- Une solution d'hypochlorite de sodium à 1% massique pendant une durée de 5 minutes

Puis rincer les fragments des feuilles ainsi stérilisées par immersion dans un bain d'eau distillée stérile à 3 reprises successives.

Cette étape a) peut également être réalisée en utilisant n'importe quelle autre partie de la plante (racines, tiges, méristèmes, fleurs...). La stérilisation peut aussi s'appliquer aux graines, sans la phase de découpe.

b) Callogenèse

On dépose des morceaux de feuilles stérilisées obtenues à l'étape précédente, sur le milieu de callogenèse solide (dont la composition est décrite dans le tableau 1), qui est ensuite incubé pendant une durée de trois semaines dans une enceinte thermostatée à une température supérieure ou égale à 20 et inférieure ou égale à25°C à la lumière ou à l'obscurité.

[Table 1]

| Composés | Concentration (mg/ L) |
|---|---|
| Glucose ou Saccharose | 30 000 |
| Acide Naphtalène Acétique | 1 |

(suite)

| Composés | Concentration (mg/ L) |
|---|---|
| 6-benzylaminopurine | 0.5 |
| Agar | 10 000 |
| $KNO_3$ | 2500 |
| $(NH_4)_2SO_4$ | 134 |
| $CaCl_2$, $2H_2O$ | 150 |
| $NaH_2PO_4$, $2H_2O$ | 150 |
| $MgSO_4$, $7H_2O$ | 250 |
| $MnSO_4$, $H_2O$ | 16,9 |
| $ZnSO_4$, $7H_2O$ | 8,6 |
| $H_3BO_3$ | 6,2 |
| KI | 0,83 |
| $Na_2MoO_4$, $2H_2O$ | 0,25 |
| $CuSO_4$, $5H_2O$ | 0,025 |
| $FeSO_4$, $7H_2O$ | 27,8 |
| $Na_2EDTA$, $2H_2O$ | 37,3 |
| Myo-inositol | 100 |
| Acide Nicotinique | 1 |
| Acide Panthotenique | 1 |
| Biotine | 0,01 |
| Pyridoxine | 1 |
| Thiamine | 1 |

composition du milieu de callogenèse

a) Mise en suspension des cellules dédifférenciées

Prélever le cal formé au cours de l'étape b) et le déposer dans un milieu liquide de mise en suspension (dont la composition est indiquée dans le tableau 2). Par « cal », on entend au sens de la présente invention un amas de cellules dédifférenciées.

Les morceaux de cal en suspension sont mis en culture dans enceinte thermostatée pendant une durée de 7 jours à 21 jours à une température supérieure ou égale à 20 et inférieure ou égale à 25°C à la lumière ou à l'obscurité, sous agitation mécanique de type orbitale, à une vitesse d'agitation comprise entre 25 tours/minute et 200 tours/minute (plus précisément à une vitesse d'agitation de 100 tours/minute pour diamètre orbital de 5 cm).

[Table 2]

| Composés | Concentration (mg/ L) |
|---|---|
| Glucose ou Saccharose | 30 000 |
| Acide Naphtalène Acétique | 1 |
| 6-benzylaminopurine | 0.5 |
| $KNO_3$ | 2500 |
| $(NH4)_2SO_4$ | 134 |
| $CaCl_2$, $2H_2O$ | 150 |

(suite)

| Composés | Concentration (mg/ L) |
|---|---|
| $NaH_2PO_4$, $2H_2O$ | 150 |
| $MgSO_4$, $7H_2O$ | 250 |
| $MnSO_4$, $H_2O$ | 16,9 |
| $ZnSO_4$, $7H_2O$ | 8,6 |
| $H_3BO_3$ | 6,2 |
| KI | 0,83 |
| $Na_2MoO_4$, $2H_2O$ | 0,25 |
| $CuSO_4$, $5H_2O$ | 0,025 |
| $FeSO_4$, $7H_2O$ | 27,8 |
| $Na_2EDTA$, $2H_2O$ | 37,3 |
| Myo-inositol | 100 |
| Acide Nicotinique | 1 |
| Acide Panthotenique | 1 |
| Biotine | 0,01 |
| Pyridoxine | 1 |
| Thiamine | 1 |

Composition du milieu de mise en suspension du cal formé à l'étape b).

b) Sélection d'une suspension fine

Prélever entre 1/5 et 1/3 du volume de culture contenant des cellules dédifférenciées en suspension fine obtenues à l'issue de l'étape c.

Déposer ce volume de suspension fine dans un volume de milieu de mise en suspension frais, trois fois supérieur au volume de suspension fine.

Cette nouvelle suspension est mise en culture dans une enceinte thermostatée pendant une durée de 7 à 21 jours (la fin de la période de culture est dictée par l'observation de la carence en nutriments) à une température supérieure ou égale à 20 et inférieure ou égale à -25°C à la lumière, sous agitation mécanique de type orbitale, à une vitesse de rotation comprise entre 25 tours/minute et 200 tours/minute (plus précisément à une vitesse de rotation de 100 tours/minute pour un diamètre orbital de 5 cm). Par « carence en nutriment », on entend au sens de la présente invention l'observation d'une teneur inférieure à 5% de la quantité de nutriment initialement introduite dans le milieu de culture.

a) Production de biomasse comprenant des cellules dédifférenciées et le milieu de culture

Prélever un volume de la suspension fine obtenue au cours de l'étape d), et le déposer dans un volume de milieu de mise en suspension frais, tel que décrit dans le tableau 2, 3 fois supérieur au volume prélevé de suspension fine obtenue à l'issu de l'étape d).

Cette nouvelle suspension est mise en culture dans une enceinte thermostatée pendant une durée de 7 à 21 jours (la fin de la période de culture est dictée par la carence en nutriments), à une température supérieure ou égale à 20 et inférieure ou égale à-25°C, à la lumière ou à l'obscurité, sous agitation mécanique de type orbitale, à une vitesse de rotation comprise entre 25 tours/minute et 200 tours/minute (plus précisément à 100 tours/minute pour diamètre orbital de 5 cm). Cette étape peut être réalisée, jusqu'à des volumes inférieurs ou égaux à 5 litres dans des Erlenmeyers, puis pour des volumes supérieurs à 5 litres et inférieurs ou égaux à 1000 Litres dans des bioréacteurs (type « réacteur à vagues » ou « wave », « réacteur à cuve agitée » ou « stired tank » ou erlenmeyer) (cette liste n'étant pas exhaustive).

b) Homogénéisation à Haute Pression de la culture : obtention du lysat de culture de cellules dédifférenciées.

Une fraction du volume de culture obtenu à l'issu de l'étape e) est prélevée pour être lysée en utilisant un homogénéisateur à haute pression, dont la pression peut être fixée à une valeur supérieur ou égale à 100 et inférieure ou égale à 1000 bars (de préférence pour la présente invention, à une pression égale à 500 bars). Les cellules dédifférenciées présentes dans la culture passée dans l'homogénéisateur haute pression sont lysées, et leur contenu est libéré dans le milieu de culture. L'efficacité d'homogénéisation (se traduisant par l'observation de débris cellulaires témoins de la lyse des cellules dédifférenciées) est facilement vérifiable par observation microscopique de la suspension.

c) Stabilisation du Lysat de culture de cellules dédifférenciées

Le lysat de culture de cellules dédifférenciées collecté à l'issu de l'étape f) est acidifié, par l'ajout d'un acide minéral ou d'un acide organique, pour atteindre une valeur de pH supérieure ou égale à 4 et inférieure ou égale à 4,5 afin de stabiliser sa couleur. Ce lysat, contient des morceaux de cellules insolubles dans le milieu et qui sont susceptibles de sédimenter, et de constituer un frein à la commercialisation du produit. L'addition d'un agent -épaississant et/ou gélifiant, dans une proportion supérieure ou égale à 0,1% massique et inférieure ou égale à 2% (plus préférentiellement un mélange de gomme de xanthane et d'acacia, dans des proportions massiques de 40-45% et 48-55% et en quantité de 0,8% massique, comme le mélange de gomme de xanthane et de gomme d'acacia commercialisé sous le nom de marque Solagum™AX) permet d'obtenir une suspension ne sédimentant pas dans le temps.

Un lysat de cellules dédifférenciées peut être stabilisé d'un point de vue microbiologique et physicochimique par différents moyens :

- Par pasteurisation (exemple: pasteurisation UHT) ,
- Par modification du pH (Exemple: acidification pH<5),
- Par addition d'un agent de réduction (Exemple: glycérine) de la disponibilité en eau,
- Par addition de conservateurs (Exemple: benzoate de sodium, sorbate de potassium), Dans notre cas, nous préférons stabiliser notre lysat par l'addition d'un mélange de glycérine, de sorbate de potassium et de benzoate de sodium.

Les proportions des différents ingrédients sont les suivantes, pour 100% massique :

- 60% à 70% massique de lysat HHP de la culture de cellules dédifférenciées d'Helichrysum stoechas, obtenu à l'étape f (95% à 99,5 % massique d'eau et 0,5% à 5% massique de matière sèche).
- 30% à 40% massique de glycérol
- 0,1% à 2% massique de Solagum™ AX (mélange de gomme d'acacia et de gomme de xanthane)
- 0,1% à 2% de sorbate de potassium
- 0,1% à 2% de benzoate de sodium

Le lysat de culture de cellules dédifférenciées stabilisé peut être stocké ou être directement incorporé à une formulation cosmétique à hauteur de 0.1% à 3% massique (préférentiellement 1% massique).

Tout type d'agent épaississant peut remplir la fonction d'agent stabilisant, par exemple Nous utilisons un solvant de nature polaire, de préférence le glycérol, mais d'autres polyols pourraient être utilisés

Notons qu'il existe bien des différences entre la plante entière et les cellules dédifférenciées. Pour montrer ces différences, nous avons effectué une comparaison. La comparaison entre la plante entière et les cellules dédifférenciées a pu être faite grâce à la comparaison d'extraits éthanoliques analysés en chromatographie liquide à haute pression (ou « HPLC »). Les échantillons sont analysés sur une colonne HPLC Kinetex C18 150*4.6 mm avec un débit de 0.8 mL/min. Les solvants sont les suivants :

- A : isopropanol à 0,05% TFA,
- B : acetonitrile à 0,05% TFA,
- C : eau à 0,05% TFA.

Le gradient est le suivant :

| t (min) | % A | %B | %C |
|---|---|---|---|
| 0 | 0 | 2 | 98 |

(suite)

| t (min) | % A | %B | %C |
|---|---|---|---|
| 5 | 0 | 2 | 98 |
| 20 | 0 | 100 | 0 |
| 25 | 0 | 100 | 0 |
| 35 | 80 | 20 | 0 |
| 40 | 80 | 20 | 0 |
| 45 | 0 | 100 | 0 |
| 50 | 0 | 2 | 98 |

Cette analyse a été réalisée avec une détection de type CAD (Charged Aerosol Détection). Les résultats sont obtenus d'un chromatogramme d'un extrait éthanolique de parties aériennes *d'Helichrysum stoechas* en détection CAD selon la méthode HPLC C18_screening et d'un chromatogramme d'un extrait éthanolique de cellules dédifférenciées d'*Helichrysum stoechas* en détection CAD selon la méthode HPLC C18_screening.

Les résultats obtenus montrent bien une différence entre les deux chromatogrammes. Le chromatogramme de l'extrait de cellules est beaucoup plus riche dans la zone entre les temps de rétention compris entre 25 minutes et 40 minutes, ce qui correspond aux temps d'élution des molécules les plus apolaires.

La présente invention a également pour objet une composition à usage topique (C1) se présentant sous la forme d'un gel et comprenant pour 100% de sa masse :

- De 95% à 99,5% massique, de préférence de 97% à 99,5% massique, encore plus préférentiellement de 97,5% à 99,5% massique du Lyzat (Ly) tel que défini précédemment et obtenu de préférence par la mise en oeuvre d'un procédé comprenant les étapes a) à f) telles que décrites ci-dessus,
- De 0,5% à 5% massique, de préférence de 0,5% à 3% massique, encore plus préférentiellement de 0,5% à 2,5% massique d'au moins un agent épaississant et/ou gélifiant.

De préférence les agents gélifiants et/ou épaississants sont choisis parmi les polysaccharides, la cellulose et les dérivés de la cellulose, les amidons, et les polymères de type polyélectrolytes, linéaires ou branchés ou réticulés.

Cette composition (C1) pourra être administrable par voie topique pour améliorer l'hydratation de la peau et/ou du cuir chevelu et/ou des lèvres.

Dans le cadre de la présente invention, on désigne par « gel » une composition chimique se présentant initialement à l'état liquide qui, après ajout de substances gélifiantes et/ou épaississantes, est transformé en un état structuré, qui ne coule pas. Un gel tel que défini, est considéré comme un état intermédiaire entre l'état solide et l'état liquide, et est constitué par un réseau tridimensionnel au sein du liquide étant le résultat de liaisons chimiques ou physiques.

Par « agent gélifiant », on entend au sens de la présente invention un composé chimique ou un mélange de composés chimiques, qui transforme un milieu liquide en un gel.

Par « agent épaississant », on entend au sens de la présente invention un composé chimique ou un mélange de composés chimiques, qui augmente la viscosité du milieu dans lequel il est introduit.

L'expression "à usage topique" utilisée dans la définition de la composition ($C_1$) telle que décrite ci-dessus, signifie que ladite composition est formulée pour permettre son application sur la peau, les cheveux, le cuir chevelu, les ongles, les lèvres, les muqueuses, les cils, les sourcils, qu'il s'agisse d'une application directe dans le cas d'une formulation cosmétique ou d'une application indirecte par exemple dans le cas d'un produit de soin corporel sous forme de lingette en textile ou en papier ou de produits sanitaires destinés à être en contact avec la peau, les cheveux, le cuir chevelu, les ongles, les lèvres, les muqueuses, les cils, les sourcils.

Dans le cadre de la présente invention, par polysaccharides, on désigne les polymères de saccharides. La définition IUPAC des saccharides désigne des oses, des composés d'oses proprement dits et leurs dérivés obtenus soit par réduction d'un groupement carbonyle, soit par oxydation d'une ou plusieurs fonctions hydroxyles, soit par le remplacement d'une ou plusieurs fonctions hydroxyles par un atome d'hydrogène, par un groupement amine, une fonction phosphate, une fonction sulfate. Les polysaccharides les plus couramment utilisés pour la préparation de compositions industrielles, alimentaires, cosmétiques ou pharmaceutiques, sont majoritairement constitués d'oses, tels que le glucose, le galactose, le mannose ou de dérivés d'oses pour lesquels la fonction hydroxyle du carbone terminal a été oxydée en fonction carboxyle. On peut distinguer parmi les polysaccharides deux groupes distincts: les polysaccharides constitués uniquement d'oses (ou poly-oses) et les polysaccharides constitués de dérivés d'oses.

Selon un aspect particulier, les agents gélifiants et/ou épaississants présents dans la composition aqueuse (C1) objet de la présente invention, sont choisis parmi les polysaccharides constitués uniquement d'oses (ou poly-oses).

Parmi les polysaccharides composés uniquement d'oses, on peut distinguer les glucanes, qui sont des homopolymères de glucose très abondants dans la nature, les glucomannoglycanes, les xyloglycanes et les galactomannanes, qui sont des polymères dont la chaîne principale est constituée d'unités de D-mannose, reliées entre elles en β-1,4, et sur laquelle des unités de D-galactose sont greffées latéralement par des liaisons α-1,6.

Les galactomannanes sont présents dans plusieurs espèces végétales, et plus particulièrement dans les espèces légumineuses dans lesquelles ils constituent l'albumen des graines. Selon leur origine végétale, le degré de substitution (DS) des unités de D-galactose sur la chaîne principale de D-mannose des galactomannanes, varie entre 0 et 1:

- les galactomannanes provenant de la gomme de cassia présentent un degré de substitution (DS) d'environ 1/5, signifiant le greffage latéral d'une unité de D-galactose toutes les 5 unités de D-mannose présentes sur la chaîne principale du polysaccharide.
- Les galactomannanes provenant de la gomme de caroube présentent un degré de substitution (DS) d'environ 1/4, signifiant le greffage latéral d'une unité de D-galactose toutes les 4 unités de D-mannose présentes sur la chaîne principale du polysaccharide
- Les galactomannanes provenant de la gomme de tara présentent un degré de substitution (DS) d'environ 1/3, signifiant le greffage latéral d'une unité de D-galactose toutes les 3 unités de D-mannose présentes sur la chaîne principale du polysaccharide.
- Les galactomannanes provenant de la gomme de guar présentent un degré de substitution (DS) d'environ 1/2, signifiant le greffage latéral d'une unité de D-galactose toutes les 2 unités de D-mannose présentes sur la chaîne principale du polysaccharide
- Les galactomannanes provenant de la gomme de fenugrec présentent un degré de substitution (DS) d'environ 1/1, signifiant le greffage latéral d'une unité de D-galactose pour quasiment toutes les unités de D-mannose présentes sur la chaîne principale du polysaccharide.

Selon un aspect plus particulier, les agents gélifiants et/ou épaississants présents dans la composition aqueuse (C1) objet de la présente invention, sont choisis parmi les polysaccharides constitués uniquement d'oses (ou poly-oses) compris dans le groupe constitué du galactomannane provenant de la gomme de Tara, du galactomannane provenant de la gomme de guar et du galactomannane provenant de la gomme de caroube. Selon un autre aspect particulier, les agents gélifiants et/ou épaississants présents dans la composition aqueuse ($C_1$) objet de la présente invention, sont choisis parmi les polysaccharides constitués de dérivés d'oses. Parmi les polysaccharides constitués de dérivés d'oses, on peut distinguer:

- Les galactanes sulfatés, qui sont des polymères de galactose pouvant avoir des groupements esters-sulfate appendus, représentés notamment par les polyosides algaux comme les carraghénanes et l'agar ;
- Les uronanes, qui sont les polymères d'acides uroniques comme les algines et les pectines ;
- Les hétéropolymères d'oses et d'acides uroniques: souvent de composition complexe, ces polymères se trouvent notamment dans les exsudats de sève (comme par exemple l'exsudat de la gomme arabique et l'exsudat de la gomme de karaya) mais ils sont produits aussi par des microorganismes, comme par exemple la gomme xanthane et la gomme gellane ;
- Les glucosaminoglycanes qui sont des polyosides formés à partir d'un glucose dérivé par remplacement de son hydroxyle sur C-2 par une amine (appelé 2-amino-2-désoxy-D-glucose ou, plus simplement, glucosamine). La fonction amine peut être d'ailleurs acétylée. Parmi les hydrocolloïdes dans cette classe on trouve le chitosane, formé uniquement de motifs glucosamine, et l'hyaluronane, dont l'unité de répétition est un dimère de glucosamine et d'acide glucuronique.

La gomme xanthane (Gx) est devenue depuis quelques décennies le polyoside microbien le plus utilisé dans l'industrie. Le xanthane est un polysaccharide synthétisé par des bactéries du genre *Xanthomonas* et, commercialement, on utilise uniquement l'espèce *X. campestris.* La chaîne principale de la (Gx) est identique à celle de la cellulose, c'est-à-dire qu'elle est formée d'unités β-D-glucose reliées par les carbones 1 et 4. On compte un triholoside branché toutes les deux unités de glucose dans la chaîne principale, de façon alternative régulière ; chaque branchement consistant en un triholoside composé de deux mannoses et d'un acide glucuronique, du type: β-D-Man*p*-(1 → 4)-β-D-GlcA*p*-(1 → 2)-α-D-Man*p*-(1 → 3) [I. Capron et al.*,"About the native and renaturated conformation of xanthan exopolysaccharide".* 1997). La gomme xanthane (GX) est disponible sous forme d'un sel de sodium, de potassium ou de calcium.

La gomme d'acacia est un polysaccharide complexe branché dont la chaîne principale consiste en des unités de β-D-galactose reliées entre elles par les carbones 1 et 3. Les chaînes branchées à la chaîne principale sont constituées

d'unités de β-D-galactose reliées entre elles par les carbones 1 et 6, portant également des unités de α-arabinose, et dans de moindres proportions des unités β-glucoronosyle. A la fois la chaîne principale et les chaînes pendantes contiennent des unités α-L-arabinosyle, α-L-rhamnopyranosyle, β-D-glucuronopyranosyle et 4-O-méthyl-β-D-glucuronopyranosyle.

Selon un aspect plus particulier, les agents gélifiants et/ou épaississants présents dans la composition aqueuse ($C_1$) objet de la présente invention sont des polysaccharides constitués de dérivés d'oses choisis parmi les éléments du groupe constitué par les carraghénanes, l'agar, les algines, les pectines, l'exsudat de la gomme arabique, l'exsudat de la gomme de karaya, la gomme de xanthane, la gomme de gellane, le chitosane et l'hyaluronane, et/ou leurs mélanges.

Selon un aspect encore plus particulier, les agents gélifiants et/ou épaississants présents dans la composition aqueuse ($C_1$) objet de la présente invention sont des polysaccharides constitués de dérivés d'oses choisis parmi les éléments du groupe constitué par l'exsudat de la gomme arabique, l'exsudat de la gomme de karaya, la gomme de xanthane et/ou leurs mélanges.

Selon un aspect encore plus particulier, les agents gélifiants et/ou épaississants présents dans la composition aqueuse ($C_1$) objet de la présente invention sont des polysaccharides constitués de dérivés d'oses choisis parmi les éléments du groupe constitué par l'exsudat de la gomme arabique, la gomme de xanthane, le mélange de gomme de xanthane (Gx) et d'exsudat de la gomme arabique ($G_A$) utilisé dans un ratio massique entre la gomme de xanthane ($G_X$) et la gomme d'exsudat d'acacia ($G_A$) est supérieur ou égal à 1/3 et inférieur ou égal à 3/1, notamment commercialisé par la société SEPPIC sous le nom de marque SOLAGUM™AX.

Selon un aspect particulier, les agents gélifiants et/ou épaississants présents dans la composition aqueuse ($C_1$) objet de la présente invention, sont choisis parmi la cellulose et les dérivés de la cellulose.

Dans le cadre de la présente invention, par « cellulose » on désigne un polysaccharide qui est constitué par une chaîne linéaire de molécules de D-Glucose, dont la masse moléculaire moyenne est d'au moins $10.000 gmol^{-1}$, plus particulièrement d'au moins $15.000 gmol^{-1}$, plus particulièrement d'au moins $17.000 gmol^{-1}$, encore plus particulièrement d'au moins $20.000 gmol^{-1}$, encore plus particulièrement d'au moins $25.000 gmol^{-1}$.

Selon un aspect plus particulier, les agents gélifiants et/ou épaississants présents dans la composition aqueuse ($C_1$) objet de la présente invention, sont choisis parmi les dérivés de cellulose.

Dans le cadre de la présente invention, par « dérivés de cellulose » on désigne les éléments du groupe constitué par l'hydroxy-éthyl cellulose, la méthyl cellulose, l'éthyl cellulose, la méthyl hydroxy éthyl cellulose, la méthyl hydroxy propyl cellulose, l' hydroxy propyl cellulose, le sel de sodium de la carboxy méthyl cellulose, le dihydroxy propyl éther de cellulose.

Dans le cadre de la présente invention, par «amidon » on désigne un mélange d'amylose et d'amylopectine, et plus particulièrement les éléments du groupe constitué par l'amidon de maïs, l'amidon de blé, l'amidon de patate et l'amidon de manioc.

Selon un aspect particulier, par «polymères de type polyélectrolytes, linéaires ou branchés ou réticulés », on désigne au sens de la présente invention:

- les copolymères anioniques synthétiques réticulés à base d'acide méthacrylique ou d'acide acrylique, ou d'esters de l'acide méthacrylique ou de l'acide acrylique, optionnellement hydrophiquement modifiés, préparés par polymérisation en émulsion directe. Ces copolymères anioniques synthétiques sont respectivement connus de l'homme du métier sous les appellations "Alcaline Swellable Emulsion" (ou "ASE") et "Hydrophobically Alcaline Swellable Emulsion" (ou "HASE"). Des agents épaississants de type "HASE" sont décrits dans la demande internationale publiée sous le numéro WO 02/34793 A2 ;
- Les polyélectrolytes anioniques synthétiques, réticulés ou branchés, qui sont des homopolymères ou des copolymères réticulés et/ou branchés de monomères insaturés hydrosolubles, comme l'acide acrylique et/ou ses dérivés, l'acide méthacrylique et/ou ses dérivés, l'acrylamide et/ou ses dérivés, l'acide 2-acrylamido-2-méthylpropanesulfonique et/ou de ses sels, la N-vinyl pyrrolidone, l'alcool vinylique et/ou ses dérivés. Ces polyélectrolytes anioniques synthétiques, réticulés ou branchés, se présentent sous la forme de latex inverses, obtenus par polymérisation radicalaire en émulsion inverse, ou sous la forme de poudres, obtenues par polymérisation précipitante ou par atomisation de latex inverses.

Selon un aspect particulier, les agents gélifiants et/ou épaississants présents dans la composition aqueuse ($C_1$) objet de la présente invention, sont choisis parmi les polyélectrolytes linéaires ou branchés ou réticulés, issus de la polymérisation radicalaire d'au moins un monomère sélectionné parmi les éléments du groupe constitué par l'acide acrylique et/ou de son sel de sodium, de l'acide méthacrylique et/ou de son sel de sodium, de l'acrylate de 2-hydroxy éthyle, du méthacrylate de 2-hydroxyéthyle, de l'acrylamide, du N,N-diméthyl acrylamide, de la N-isopropyl acrylamide, l'acide 2-acrylamido-2-méthylpropanesulfonique et/ou son sel de sodium ou de potassium, la N-vinyl pyrrolidone, au moins un monomère de formule (I) :

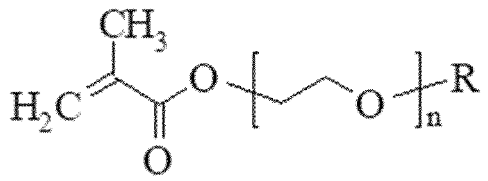

(I)

dans laquelle R représente un radical alkyle linéaire ou ramifié comportant de huit à vingt atomes de carbone et n représente un nombre entier supérieur ou égal à zéro et inférieur ou égal à vingt. Ladite polymérisation radicalaire est conduite en présence d'un agent réticulant choisi parmi les monomères polyéthyléniques comprenant au moins deux fonctions éthyléniques, et plus particulièrement choisi parmi les éléments du groupe constitué par le diméthacrylate d'éthylèneglycol, le tétraallyloxyéthane, le diacrylate d'éthylèneglycol, le diallyl urée, le triallyl amine, le triméthylol propanetriacrylate ou le méthylène-bis(acrylamide) ou un mélange de ces composés.

Selon un aspect particulier, les agents gélifiants et/ou épaississants présents dans la composition aqueuse ($C_1$) objet de la présente invention, sont choisis parmi les éléments du groupe constitué par:

- L'homopolymère de l'acide acrylique partiellement ou totalement salifié, réticulé au triallyl amine et/ou au triméthylol propanetriacrylate et/ou au méthylène-bis(acrylamide),
- L'homopolymère du sel de sodium de l'acide 2-acrylamido 2-méthyl propanesulfonique, réticulé au triallyl amine et/ou au triméthylol propanetriacrylate et/ou au méthylène-bis(acrylamide),
- Le copolymère du sel de sodium de l'acide 2-acrylamido 2-méthyl propanesulfonique et de l'acide acrylique partiellement ou totalement salifié, réticulé au triallyl amine et/ou au triméthylol propanetriacrylate et/ou au méthylène-bis(acrylamide),
- Le copolymère du sel de sodium de l'acide 2-acrylamido 2-méthyl propanesulfonique et de l'acrylate de 2-hydroxy éthyle, réticulé au triallyl amine et/ou au triméthylol propanetriacrylate et/ou au méthylène-bis(acrylamide),
- Le copolymère du sel de sodium de l'acide 2-acrylamido 2-méthyl propanesulfonique et de l'acrylamide, réticulé au triallyl amine et/ou au triméthylol propanetriacrylate et/ou au méthylène-bis(acrylamide),
- Le terpolymère du sel de sodium de l'acide 2-acrylamido 2-méthyl propanesulfonique, de l'acrylamide et l'acide acrylique partiellement ou totalement salifié, réticulé au triallyl amine et/ou au triméthylol propanetriacrylate et/ou au méthylène-bis(acrylamide),
- Le terpolymère du sel de sodium de l'acide 2-acrylamido 2-méthyl propanesulfonique, du N,N-diméthylacrylamide, et l'acide acrylique partiellement ou totalement salifié, réticulé au triallyl amine et/ou au triméthylol propanetriacrylate et/ou au méthylène-bis(acrylamide),
- un terpolymère réticulé par le trimethylolpropanetriacrylate et/ou le triallylamine et/ou le méthylène-bis(acrylamide), de l'acide 2-méthyl 2-[(1-oxo 2- propènyl) amino] 1-propanesulfonique partiellement ou totalement salifié sous forme de sel de sodium ou de sel d'ammonium dans une proportion molaire supérieure ou égale à 60% et inférieure ou égale à 80%, du N,N diméthylacrylamide dans une proportion molaire supérieure ou égale à 15% et inférieure ou égale à 39,5% et du méthacrylate de lauryle tétraéthoxylé dans une proportion molaire supérieure ou égale à 0,5% et inférieure ou égale à 5%,
- un tétrapolymère réticulé par le trimethylolpropanetriacrylate et/ou le triallylamine et/ou le méthylène-bis(acrylamide), de l'acide 2-méthyl 2-[(1-oxo 2- propènyl) amino] 1-propanesulfonique partiellement ou totalement salifié sous forme de sel de sodium ou de sel d'ammonium dans une proportion molaire supérieure ou égale à 60% et inférieure ou égale à 80%, du N,N diméthylacrylamide dans une proportion molaire supérieure ou égale à 15% et inférieure ou égale à 39 %, du méthacrylate de lauroyle dans une proportion molaire supérieure ou égale à 0,5% et inférieure ou égale à 2,5%, et du méthacrylate de stéaroyle dans une proportion molaire supérieure ou égale à 0,5% et inférieure ou égale à 2 ,5%.

Selon un aspect plus particulier, les agents gélifiants et/ou épaississants présents dans la composition aqueuse ($C_1$) objet de la présente invention, sont choisis parmi les éléments du groupe constitué par la gomme de xanthane, l'exsudat de la gomme d'acacia, le mélange de gomme de xanthane ($G_X$) et d'exsudat de la gomme arabique ($G_A$) dans un ratio massique entre la gomme de xanthane ($G_X$) et la gomme d'exsudat d'acacia ($G_A$) est supérieur ou égal à 1/3 et inférieur ou égal à 3/1, le copolymère du sel de sodium de l'acide 2-acrylamido 2-méthyl propanesulfonique et de l'acrylate de 2-hydroxy éthyle, réticulé au triallyl amine et/ou au triméthylol propanetriacrylate et/ou au méthylène-bis(acrylamide), le copolymère du sel de sodium de l'acide 2-acrylamido-2-méthyl propanesulfonique et de l'acrylamide, réticulé au triallyl amine et/ou au triméthylol propanetriacrylate et/ou au méthylène-bis(acrylamide), le terpolymère réticulé par le trimethylolpropanetriacrylate et/ou le triallylamine et/ou le méthylène-bis(acrylamide), de l'acide 2-méthyl 2-[(1-oxo 2- propènyl) amino] 1-propanesulfonique partiellement ou totalement salifié sous forme de sel de sodium ou de sel

d'ammonium dans une proportion molaire supérieure ou égale à 60% et inférieure ou égale à 80%, du N,N diméthylacrylamide dans une proportion molaire supérieure ou égale à 15% et inférieure ou égale à 39,5% et du méthacrylate de lauryle tétraéthoxylé dans une proportion molaire supérieure ou égale à 0,5% et inférieure ou égale à 5%.

La composition à usage topique ($C_1$) telle que définie précédemment, peut comprendre en outre, un ou plusieurs composés auxiliaires choisis parmi les solvants et cosolvants, les agents d'amélioration de la pénétration cutanée.

La présente invention a également pour objet une composition à usage topique (C'1) se présentant sous la forme d'un gel et comprenant pour 100% de sa masse :

- De 50% à 80% massique, de préférence de 50 à 75% massique, et encore plus préférentiellement de 60 à 70% massique du Lyzat (Ly) tel que défini précédemment,
- De 0,1% à 5% massique, de préférence de 0,5% à 3% massique d'au moins un agent épaississant et/ou gélifiant, et
- De 15% à 49,9% massique, de préférence de 22% à 49,5% massique, et encore plus préférentiellement de 27% à 39,5% massique d'au moins un solvant.

De préférence, le solvant est choisi parmi les éléments du groupe constitué par :

- les composés de formule (Ia) :

  HO-[CH2-CH(OH)-CH2-O]n-H (Ia)

  dans laquelle n représente un nombre entier supérieur ou égal à un et inférieur ou égal à quinze, plus particulièrement supérieur ou égal à un et inférieur ou égal à 10, plus particulièrement supérieur ou égal à un et inférieur ou égal à 6, plus particulièrement supérieur ou égal à un et inférieur ou égal à 4, plus particulièrement égal à 1 ou 2 ou 3 ou 4 ;
- les composés de formule (Ib) :

  Ra1-C(Rb1)(OH)-C(OH)(Rc1)(Rd1) (Ib),

  dans laquelle chacun des radicaux Ra1, Rb1, Rc1 et Rd1 représentent indépendamment les uns des autres, un atome d'hydrogène ou radical aliphatique saturé comportant de un à cinq atomes de carbone, soit par la formule (Ib1) :

  Ra1-C(Rb1)(OH)-[C(Re1)(Rf1)]t-C(OH)(Rc1)(Rd1) (Ib1),

  dans laquelle t est égal à un, deux ou trois, chacun des radicaux Ra1, Rb1, Rc1, Rd1, Re1 et Rf1 représentent indépendamment les uns des autres, un atome d'hydrogène ou radical aliphatique saturé comportant de un à cinq atomes de carbone, étant entendu que l'un au moins des radicaux Ra1 ou Rb1 et/ou l'un au moins des radicaux Rc1 ou Rd1 ne représentent pas un atome d'hydrogène.

Encore plus préférentiellement le solvant sera du glycérol.

Cette composition (C'1) pourra être administrable par voie topique pour améliorer l'hydratation de la peau et/ou du cuir chevelu et/ou des lèvres.

Les compositions à usage topique ($C_1$) et ($C'_1$) telles que définies précédemment, peuvent comprendre en outre, un ou plusieurs cosolvants, et agents d'amélioration de la pénétration cutanée.

Comme exemples de co-solvants éventuellement présents dans les compositions à usage topique ($C_1$) et ($C'_1$) objet de la présente invention, on peut citer l'eau, les solvants organiques par exemple le glycérol, le diglycérol, les oligomères du glycérol, l'éthylène glycol, le propylène glycol, le butylène glycol, l'hexylène glycol, le diéthylène glycol, le xylitol, l'érythritol, le sorbitol, les alcools hydrosolubles tels que l'éthanol, l'isopropanol ou le butanol, les mélanges d'eau et desdits solvants organiques, le propylène carbonate, l'acétate d'éthyle, l'alcool benzylique.

Comme exemples d'agents d'amélioration de la pénétration cutanée éventuellement présents dans les compositions à usage topique ($C_1$) et ($C'_1$) objet de la présente invention, on peut citer les glycols éthers comme par exemple l'éthylène glycol mono méthyl éther, l'éthylène glycol mono éthyl éther, l'éthylène glycol mono propyl éther, l'éthylène glycol mono isopropyl éther, l'éthylène glycol mono butyl éther, l'éthylene glycol mono phényl éther, l'éthylène glycol mono benzyl éther, le diéthylène glycol mono méthyl éther, le diéthylène glycol mono éthyl éther et le diéthylène glycol mono-n-butyl éther, le diéthylène glycol mono éthyléther (ou Transcutol-P), les acides gras comme l'acide oléique, les esters de glycérol d'acide gras comme par exemple le béhénate de glycérol, le palmitostéarate de glycérol, le béhénoyl macroglycérides, le polyoxyéthylène-2-stéaryl éther, le polyoxyéthylène-2-oléyl éthers, des terpènes comme par exemple le D-Limonène, des huiles essentielles comme par exemple l'huile essentielle d'eucalyptus.

La présente invention a également pour objet une composition à usage topique (C2) se présentant sous la forme d'une émulsion de type eau-dans-huile ou d'une émulsion de type huile-dans-eau, comprenant le lysat (Ly) tel que défini précédemment, et obtenu de préférence par la mise en oeuvre d'un procédé comprenant les étapes a) à f) telles que décrites ci-dessus.

On distingue les émulsions huile-dans-eau (H/E) dont la phase continue est constituée par une phase hydrophile, généralement une phase aqueuse, et la phase dispersée par une phase grasse lipophile, et les émulsions eau-dans-huile (E/H) dont la phase continue est constituée par une phase grasse lipophile et la phase dispersée par une phase hydrophile, généralement une phase aqueuse.

Cette composition (C2) pourra également être administrable par voie topique pour améliorer l'hydratation de la peau et/ou du cuir chevelu et/ou des lèvres.

La composition (C2) pourra en particulier être administrable par voie topique pour diminuer et/ou éliminer et/ou prévenir les gerçures et/ou les dartres et/ou les crevasses et/ou la dermatite atopique et/ou l'ichtyose et/ou des états de sécheresse de la peau ou des muqueuses, accompagnant des pathologies cutanées et/ou mucosales telles que l'eczéma. Parmi les émulsions de type eau-dans-huile , la présente invention a pour objet particulier une composition à usage topique (F) se présentant sous la forme d'une émulsion eau-dans-huile et comprenant pour 100% de sa masse :

- de 60% à 90% massique de la composition à usage topique (C1) ou (C'1) telle que définie à l'une des revendications 3 à 9,
- de 10 % à 40 % massique d'une phase grasse ($A_2$) comprenant i) au moins une huile, et optionnellement au moins une cire et ii) un système émulsionnant comprenant au moins un agent tensioactif émulsionnant ($S_1$).

De préférence, l'agent tensioactif émulsionnant ($S_1$) sera choisi parmi les éléments du groupe constitué par les compositions d'alkylpolyglycosides, les compositions d'alkylpolyglycosides et d'alcools gras, les esters de polyglycérols, les esters de polyglycérols alcoxylés, les polyhydroxystéarates de polyglycols, les polyhydroxystéarates de polyglycérols, les polyhydroxystéarates de polyglycérols alcoxylés.

Dans la formulation (F) à usage topique objet de la présente invention, on désigne par « huile » un composé et/ou un mélange de composés insoluble dans l'eau, et liquide à 25°C, et plus particulièrement :

- Les alcanes linéaires comportant de onze à dix-neuf atomes de carbone ;
- Les alcanes ramifiés, comportant de sept à quarante atomes de carbone, comme l'isododécane, l'isopentadécane, l'isohexadécane, l'isoheptadécane, l'isooctadécane, l'isononadécane ou l'isoeicosane), ou des mélanges de certains d'entre eux comme ceux cités ci-après et identifiés par leur nom INCI : $C_{7-8}$ isoparaffin, $C_{8-9}$ isoparaffin, $C_{9-11}$ isoparaffin, $C_{9-12}$ isoparaffin, $C_{9-13}$ isoparaffin, $C_{9-14}$ isoparaffin, $C_{9-16}$ isoparaffin, $C_{10-11}$ isoparaffin, $C_{10-12}$ isoparaffin, $C_{10-13}$ isoparaffin, $C_{11-12}$ isoparaffin, $C_{11-13}$ isoparaffin, $C_{11-14}$ isoparaffin, $C_{12-14}$ isoparaffin, $C_{12-20}$ isoparaffin, $C_{13-14}$ isoparaffin, $C_{13-16}$ isoparaffin ;
- Les cyclo-alcanes optionnellement substitués par un ou plusieurs radicaux alkyles linéaires ou ramifiés ;
- Les huiles blanches minérales, comme celles commercialisées sous les noms suivants : Marcol™52, Marcol™82, Drakeol™6VR, Eolane™130, Eolane™150 ;
- L'hémisqualane (ou 2,6,10-trimethyl- dodécane ; numéro CAS : 3891-98-3), le squalane (ou 2,6,10,15,19,23-hexamethyltetracosane), le polyisobutène hydrogéné ou le polydécène hydrogéné ;
- Les mélanges d'alcanes comportant de 15 à 19 atomes de carbone, lesdits alcanes étant des alcanes linéaires, des alcanes ramifiés et des cyclo-alcanes, et plus particulièrement le mélange ($M_1$) qui comprend pour 100% de sa masse, une proportion massique en alcanes ramifiés supérieure ou égale à 90% et inférieure ou égale à 100% ; une proportion massique en alcanes linéaires supérieure ou égale à 0% et inférieure ou égale à 9%, et plus particulièrement inférieure à 5% et une proportion massique en cyclo-alcanes supérieure ou égale à 0% et inférieure ou égale à 1%, par exemple les mélanges commercialisés sous les noms Emogreen™L15 ou Emogreen™L19 ;
- Les éthers d'alcool gras de formule (II) :

$$Z_1\text{-O-}Z_2 \qquad \text{(II)},$$

  dans laquelle $Z_1$ et $Z_2$ identiques ou différents, représentent un radical alkyle linéaire ou ramifié comportant de cinq à dix-huit atomes de carbone, par exemple les dioctyl éther, didécyl éther, didodécyl éther, dodécyl octyl éther, dihexadécyl éther, (1,3-diméthyl butyl) tétradécyl éther, (1,3-diméthyl butyl) hexadécyl éther, le bis(1,3-diméthyl butyl) éther ou le dihexyl éther.
- Les mono-esters d'acides gras et d'alcools de formule (III) :

$$R'_1\text{-(C=O)-O-}R'_2 \qquad \text{(III)},$$

dans laquelle $R'_1$-(C=O) représente un radical acyle, saturé ou insaturé, linéaire ou ramifié, comportant de huit à vingt-quatre atomes de carbone, et R'2 représente, indépendamment de $R'_1$, une chaîne hydrocarbonée saturée ou insaturée, linéaire ou ramifiée comportant de un à vingt-quatre atomes de carbone, par exemple les laurate de méthyle, laurate d'éthyle, laurate de propyle, laurate d'isopropyle, laurate de butyle, laurate de 2-butyle, laurate d'hexyle, cocoate de méthyle, cocoate d'éthyle, cocoate de propyle, cocoate d'isopropyle, cocoate de butyle, cocoate de 2-butyle, cocoate d'hexyle, myristate de méthyle, myristate d'éthyle, myristate de propyle, le myristate d'isopropyle, le myristate de butyle, le myristate de 2-butyle, le myristate d'hexyle, le myristate d'octyle, le palmitate de méthyle, le palmitate d'éthyle, le palmitate de propyle, le palmitate d'isopropyle, le palmitate de butyle, le palmitate de 2-butyle, le palmitate d'hexyle, le palmitate d'octyle , l'oléate de méthyle, l' oléate d'éthyle, l'oléate de propyle, l'oléate d'isopropyle, l'oléate de butyle, l'oléate de 2-butyle, l'oléate d'hexyle, l'oléate d'octyle, le stéarate de méthyle, le stéarate d'éthyle, le stéarate de propyle, le stéarate d'isopropyle, le stéarate de butyle, le stéarate de 2-butyle, le stéarate d'hexyle, le stéarate d'octyle, l'isostéarate de méthyle, l'isostéarate d'éthyle, l'isostéarate de propyle, l'isostéarate d'isopropyle, l'isostéarate de butyle, l'isostéarate de 2-butyle, l'isostéarate d'hexyle, l'isostéarate d'isostéaryle ;

- Les di-esters d'acides gras et de glycérol de formule (IV) et de formule (V) :

$$R'_3\text{-(C=O)-O-}CH_2\text{-CH(OH)-}CH_2\text{-O-(C=O)-}R'_4 \qquad (IV)$$

$$R'_5\text{-(C=O)-O-}CH_2\text{-CH [O-(C=O)-}R'_{6]}\text{-}CH_2\text{-OH} \qquad (V),$$

formules (VI) (VII) dans lesquelles $R'_3$-(C=O), $R'_4$-(C=O), $R'_5$-(C=O), $R'_6$-(C=O), identiques ou différents, représentent un groupement acyle, saturé ou insaturé, linéaire ou ramifié, comportant de huit à vingt-quatre atomes de carbone ;

- Les tri-esters d'acides gras et de glycérol de formule (VI) :

$$R'_7\text{-(C=O)-O-}CH_2\text{-CH[O-(C=O)-}R''_8\text{]-}CH_2\text{-O-(C=O)-}R''_9 \qquad (VI),$$

dans laquelle $R'_7$-(C=O), R's-(C=O) et $R'_9$-(C=O), identiques ou différents, représentent un groupement acyle, saturé ou insaturé, linéaire ou ramifié, comportant de huit à vingt-quatre atomes de carbone.

- Les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;
- Les huiles végétales éthoxylées.

De préférence, la composition selon l'invention comprend au moins une huile choisie parmi les éléments du groupe constitué par l'huile de ricin, les huiles de paraffine, le cocoyl caprylate/caprate, le myristate d'isopropyle, le capric caprylic triglycéride.

La phase grasse (A2) comprend optionnellement de la cire. Dans la formulation (F) à usage topique objet de la présente invention, on désigne par « cire » un composé et/ou un mélange de composés insoluble dans l'eau, et solide à 35°C

Une telle cire est plus particulièrement choisie parmi la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline; l'ozokérite; la cire de polyéthylène; les cires de silicone ; les cires végétales ; les alcools gras et les acides gras solides à température ambiante; les glycérides solides à température ambiante.

Dans la définition de la formulation (F) objet de la présente invention, on désigne par « compositions d'alkylpolyglycosides », une composition (A) représentée par la formule (VII)

$$R_1\text{-O-(G)}_x\text{-H} \qquad (VII)$$

dans laquelle x représente un nombre décimal compris entre 1,05 et 5, G représente le reste d'un sucre réducteur, et $R_1$ représente un radical aliphatique hydrocarboné, saturé ou insaturé, linéaire ou ramifié, éventuellement substitué avec un ou plusieurs groupe hydroxyle, comportant de 12 à 36 atomes de carbone, ladite composition (A) consistant en un mélange de composés représentés par les formules ($VII_1$), ($VII_2$), ($VII_3$), ($VII_4$) et ($VII_5$) :

$R_1$-O-$(G)_1$-H (VII$_1$)

$R_1$-O-$(G)_2$-H (VII$_2$)

$R_1$-O-$(G)_3$-H (VII$_3$)

$R_1$-O-$(G)_4$-H (VII$_4$)

$R_1$-O-$(G)_5$-H (VII$_5$)

dans les proportions molaires respectives $a_1$, $a_2$, $a_3$, $a_4$ et $a_5$, telles que :

- La somme $a_1$+ $a_2$ + $a_3$ + $a_4$ + $a_5$ est égale à 1 et que
- La somme $a_1$ + 2az + $3a_3$ + $4a_4$ + 5as est égale à x.

Par radical aliphatique hydrocarboné, saturé ou insaturé, linéaire ou ramifié, comportant de 12 à 36 atomes de carbone, éventuellement substitué avec un ou plusieurs groupes hydroxyle, on désigne pour le radical $R_1$ dans la formule (VII) telle que définie ci-dessus :

- Les radicaux alkyle linéaires saturés, par exemple les radicaux n-dodécyle, n-tridécyle, n-tétradécyle, n-pentadécyle, n-hexadécyle, n-heptadécyle, n-octadécyle, n-nonadécyle, n-eicosyle, n-docosyle ;
- Les radicaux linéaires insaturés tels que les radicaux dodécènyle, tridécènyle, tétradécènyle, pentadécènyle, hexadécènyle, heptadécènyle, octadécènyle, nonadécènyle, eicosènyle, docosènyle, 4-dodécènyle, ou 5-dodécènyle ;
- Les radicaux aliphatiques saturés ou insaturés, linéaires ou ramifiés, comportant de 12 à 36 atomes de carbone substitués par un ou deux groupes hydroxy, tels que les radicaux hydroxydodécyle, hydroxytétradécyle, hydroxyhexadécyle, hydroxyoctadécyle, hydroxyeicosyle, hydroxydocosyle, par exemple le radical 12-hydroxy octadécyle.
- Les radicaux issus des isoalcanols de formule (1) :

$$(CH_3)(CH_3)CH-(CH_2)_r-CH_2-OH \quad (1)$$

dans laquelle r représente un nombre entier compris entre 8 et 20, par exemple les radicaux isodécyle, isoundécyle, isododécyle, isotridécyle, isotétradécyle, isopentadécyle, isohexadécyle, isopentadécyle, isooctadécyle, isononadécyle, isoeicosyle ou isodocosyle ; Les radicaux alkyles ramifiés, issus des alcools de Guerbet, de formule (2) :

$$CH(C_sH_{2s+1})(C_tH_{2t+1})-CH_2-OH \quad (2)$$

dans laquelle t est un nombre entier compris entre 6 et 18, s est un nombre entier compris entre 4 et 18 et la somme s + t est supérieure ou égale à 10, et inférieure ou égale à 22, par exemple les radicaux 2-butyl octyle, 2-butyl décyle, 2-hexyl octyle, 2-hexyl décyle, 2-octyl décyle, 2-hexyl dodécyle, 2-octyl dodécyle, 2-décyl tétradécyle, 2-dodécyl hexadécyle, 2-tétradécyl octadécyle.

Selon un aspect particulier, dans la définition de la formule (VII) telle que définie ci-dessus, $R_1$ représente un radical aliphatique hydrocarboné, saturé ou insaturé, linéaire ou ramifié comportant de 12 à 24 atomes de carbone.

Par sucre réducteur, dans la définition de la formule (VII) telle que définie ci-dessus, on désigne les dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990. La structure oligomérique $(G)_x$, peut se présenter sous toutes formes d'isoméries, qu'il s'agisse d'isomérie optique, d'isomérie géométrique ou d'isomérie de position ; elle peut aussi représenter un mélange d'isomères.

Dans la formule (VII) telle que définie ci-dessus, le groupe $R_1$-O- est lié à G par le carbone anomérique du reste saccharide, de manière à former une fonction acétal.

Selon un aspect particulier dans la définition de la formule (VII) telle que définie ci-dessus, G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane ou le tallose; et plus particulièrement G représente le reste d'un sucre réducteur choisi

parmi les restes du glucose, du xylose et de l'arabinose.

Selon un aspect encore plus particulier, dans la définition de la formule (VII), x représente un nombre décimal supérieur ou égal à 1,05 et inférieur ou égal à 2,5, plus particulièrement supérieur ou égal à 1,05 et inférieur ou égal à 2,0, et encore plus particulièrement supérieur ou égal à 1,25 et inférieur ou égal à 2,0.

Selon un aspect encore plus particulier, dans la définition de la formule (VII) telle que définie ci-dessus, $R_1$ représente le radical choisi parmi au moins un des éléments du groupe constitué par les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle, 2-décyl tétradécyle ; G représente le reste d'un sucre réducteur choisi parmi les restes du glucose et du xylose et x représente un nombre décimal supérieur ou égal à 1,05 et inférieur ou égal à 2,5.

Selon un aspect encore plus particulier, la formulation (F) telle que définie précédemment, est caractérisée en ce que ledit système émulsionnant consiste en une composition (A) d'alkylpolyglycosides représentée par la formule (VII) :

$R_1$-O-$(G)_x$-H (VII)

dans laquelle x représente un nombre décimal compris entre 1,05 et 2,5, G représente le reste du xylose, et $R_1$ représente le radical 2-octyl dodécyle, ladite composition ($C_2$) consistant en un mélange de composés représentés par les formules ($VII_1$), ($VII_2$), ($VII_3$), ($VII_4$) et ($VII_5$) :

$R_1$-O-$(G)_1$-H ($VII_1$)

$R_1$-O-$(G)_2$-H ($VII_2$)

$R_1$-O-$(G)_3$-H ($VII_3$)

$R_1$-O-$(G)_4$-H ($VII_4$)

$R_1$-O-$(G)_5$-H ($VII_5$)

dans les proportions molaires respectives $a_1$, $a_2$, $a_3$, $a_4$ et $a_5$, telles que :

- La somme $a_1 + a_2 + a_3 + a_4 + a_5$ est égale à 1 et que
- La somme $a_1 + 2a_2 + 3a_3 + 4a_4 + 5a_5$ est égale à x.

Dans la définition de la composition (F) objet de la présente invention, on désigne par composition d'alkylpolyglycosides et d'alcools gras, une composition (B) comprenant pour 100% de sa masse :

- De 10% à 50% massique, plus particulièrement de 15% à 40% massique, et encore plus particulièrement de 20% à 30% massique, d'au moins une composition (A) représentée par la formule (VII) telle que définie précédemment,
- De 90% à 50% massique, plus particulièrement de 85% à 60% massique, et encore plus particulièrement de 80% à 70% massique, d'au moins un alcool gras de formule (VIII) :
- $R'_1$-OH (VIII),

dans laquelle $R'_1$, identique ou différent de $R_1$, représente un radical aliphatique hydrocarboné, saturé ou insaturé, linéaire ou ramifié, éventuellement substitué avec un ou plusieurs groupe hydroxyle, comportant de 12 à 36 atomes de carbone.

Selon un aspect particulier, dans la définition de la formule (VII) représentant la composition (A) comprise dans la composition (B), $R_1$ représente le radical choisi parmi les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle, 2-décyl tétradécyle, G représente le reste d'un sucre réducteur choisi parmi les restes du glucose et du xylose et x représente un nombre décimal supérieur ou égal à 1,05 et inférieur ou égal à 2,5.

Selon un aspect plus particulier, dans la définition de la formule (VII) représentant la composition (A) comprise dans la composition (B), $R_1$ représente le radical 2-octyl dodécyle, G représente le reste du xylose et x représente un nombre décimal supérieur ou égal à 1,05 et inférieur ou égal à 2,5.

Selon un aspect plus particulier, dans la définition de l'alcool gras de formule (VIII) telle que définie ci-dessus, $R'_1$ représente un radical choisi parmi les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle ou 2-décyl tétradécyle, $R'_1$ représente

tout particulièrement le radical 2-octyl dodécyle.

Selon un aspect encore plus particulier, l'invention a pour objet une formulation (F) telle que définie précédemment caractérisée en ce que ledit système émulsionnant consiste en une composition (B) comprenant pour 100% de sa masse :

- De 10% à 50% massique d'au moins une composition d'alkylpolyglycosides (A) représentée par la formule (VII) :

$R_1$-O-$(G)_x$-H (VII)

dans laquelle x représente un nombre décimal compris entre 1,05 et 2,5, G représente le reste du xylose et $R_1$ représente le radical 2-octyl dodécyle, ladite composition consistant en un mélange de composés représentés par les formules ($VII_1$), (VIIz), ($VII_3$), ($VII_4$) et ($VII_5$) :

$R_1$-O-$(G)_1$-H ($VII_1$)

$R_1$-O-$(G)_2$-H ($VII_2$)

$R_1$-O-$(G)_3$-H ($VII_3$)

$R_1$-O-$(G)_4$-H ($VII_4$)

$R_1$-O-$(G)_5$-H ($VII_5$)

dans les proportions molaires respectives $a_1$, $a_2$, $a_3$, $a_4$ et $a_5$, telles que :

- La somme $a_1 + a_2 + a_3 + a_4 + a_5$ est égale à 1 et que
- La somme $a_1 + 2a_2 + 3a_3 + 4a_4 + 5a_5$ est égale à x ; et
- De 90% à 50% massique d'au moins un alcool gras de formule (VIII) :

$R'_1$-OH (VIII),

dans laquelle $R'_1$ représente le radical 2-octyl dodécyle.

Dans la définition de la formulation (F) objet de la présente invention, on désigne par ester de polyglycérol, un composé de formule (IX) :

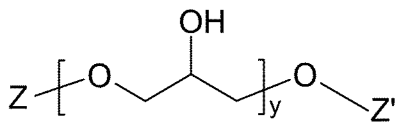

(IX)

dans laquelle Z représente un radical acyle de formule $R_2$-C(=O)-, dans laquelle $R_2$ représente un radical aliphatique hydrocarboné, saturé ou insaturé, linéaire ou ramifié, comportant de 11 à 35 atomes de carbone et plus particulièrement un radical choisi parmi les radicaux dodécanoyle, tétradécanoyle, hexadécanoyle, octadécanoyle, eicosanoyle, docosanoyle, oléyle, linoléyle, linolénoyle ou isostéaryle, Z' représente le radical acyle de formule $R_2$-C(=O)-tel que défini ci-dessus, avec Z' identique ou différent de Z, ou l'atome d'hydrogène, et y représente un nombre entier supérieur ou égal à 2 et inférieur ou égal à 20.

Selon un aspect plus particulier, le composé de formule (IX) est choisi parmi les éléments du groupe constitué par l'oléate de décaglycérol, l'isostéarate de décaglycérol, le monolaurate de décaglycérol, le mono-linoléate de décaglycérol, le mono-myristate de décaglycérol.

Dans la définition de la formulation (F) objet de la présente invention, on désigne par esters de polyglycérols alcoxylés, un composé de formule (X) :

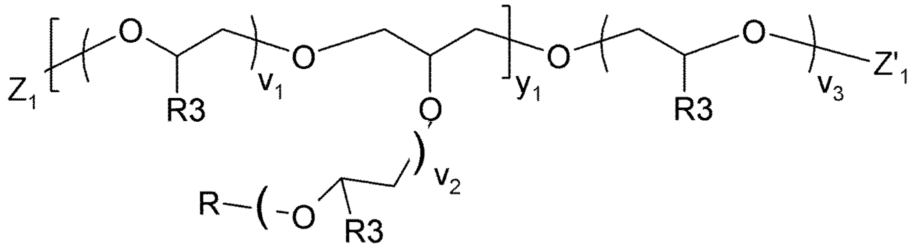

(X)

dans laquelle $Z_1$ représente un radical acyle de formule $R'_2$-C(=O)-, dans laquelle $R'_2$ représente un radical aliphatique hydrocarboné, saturé ou insaturé, linéaire ou ramifié, comportant de 11 à 35 atomes de carbone, et plus particulièrement un radical choisi parmi les radicaux radicaux dodécanoyle, tétradécanoyle, hexadécanoyle, octadécanoyle, eicosanoyle, docosanoyle, oléyle, linoléyle, linolénoyle ou isostéaryle, $Z_1$' représente le radical acyle de formule $R'_2$-C(=O)- tel que défini ci-dessus, avec $Z_1$' identique ou différent de $Z_1$, ou l'atome d'hydrogène, $R_3$ représente l'atome d'hydrogène, le radical méthyle, ou le radical éthyle, $y_1$ représente un nombre entier supérieur ou égal à 2 et inférieur ou égal à 20, $v_1$, $v_2$, $v_3$, identiques ou différents, représentent un nombre entier supérieur ou égal à 0 et inférieur ou égal à 50, et la somme $[(y_1 . v_1) + (y_1 . v_2) + v_3)]$ est un nombre entier supérieur ou égal à 1 et inférieur ou égal à 50.

Dans la définition de la composition (F) objet de la présente invention, on désigne par polyhydroxystéarates de polyglycols, un composé de formule (XI) :

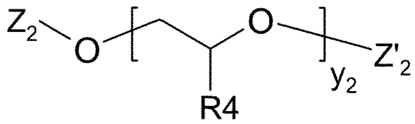

(XI)

dans laquelle yz représente un nombre entier supérieur ou égal à 2 et inférieur ou égal à 50, $R_4$ représente l'atome d'hydrogène, le radical méthyle, ou le radical éthyle, $Z_2$ représente un radical de formule (XII) :

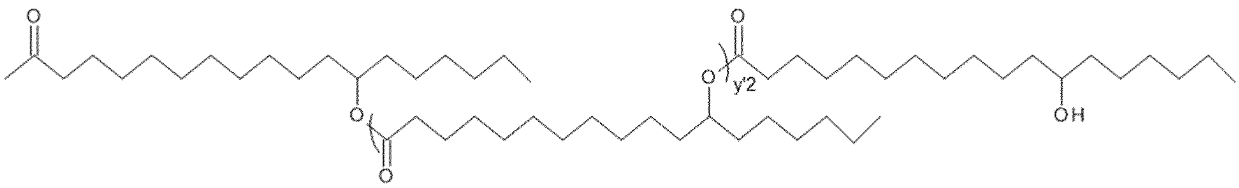

(XII),

dans laquelle $y'_2$ représente un nombre entier supérieur ou égal à 0 et inférieur ou égal à 10, plus particulièrement supérieur ou égal à 1 et inférieur ou égal à 10 et $Z'_2$ représente un radical de formule (XII) telle que définie ci-dessus, avec $Z_2$' identique ou différent de $Z_2$, ou l'atome d'hydrogène.

Dans la définition de la formulation (F) objet de la présente invention, on désigne par polyhydroxystéarate de polyglycérol, un composé représenté par la formule (XIII) :

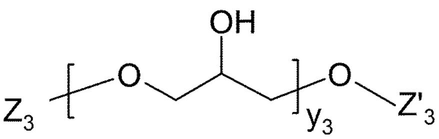

(XIII)

dans laquelle $Z_3$ représente un radical de formule (XII) telle que définie ci-dessus, $Z'_3$ représente un radical de formule (XII) telle que définie ci-dessus, avec $Z_3$' identique ou différent de $Z_3$, ou l'atome d'hydrogène, $y_3$ représente un nombre entier supérieur ou égal à 2 et inférieur ou égal à 20.

Dans la définition de la formulation (F) objet de la présente invention, on désigne par polyhydroxystéarate de polyglycérol alcoxylé, un composé représenté par la formule (XIV) :

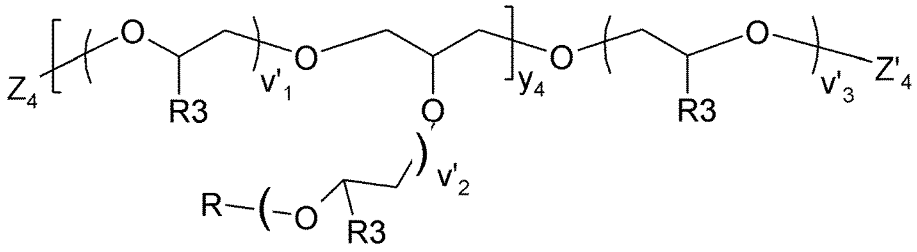

(XIV),

dans laquelle $Z_4$ représente un radical de formule (XII) telle que définie ci-dessus, $Z'_4$ représente un radical de formule (XII) telle que définie ci-dessus, avec $Z_4'$ identique ou différent de $Z_4$, ou l'atome d'hydrogène, $y_4$ représente un nombre entier supérieur ou égal à 2 et inférieur ou égal à 20, $v'_1$, $v'_2$, $v'_3$, identiques ou différents, représentent un nombre entier supérieur ou égal à 0 et inférieur ou égal à 50, et la somme $[(y_4 . v'_1) + (y_4 . v'_2) + v'_3)]$ est un nombre entier supérieur ou égal à 1 et inférieur ou égal à 50.

Selon un autre aspect particulier, l'invention a pour objet une formulation (F) telle que définie précédemment, caractérisée en ce que ledit système émulsionnant (S) consiste en une composition (D) comprenant pour 100% de sa masse :

De 15% à 25% massique d'au moins une composition (A) représentée par la formule (VII) :

$R1\text{-}O\text{-}(G)_x\text{-}H$ (VII)

dans laquelle x représente un nombre décimal compris entre 1,05 et 2,5, G représente le reste du xylose et $R_1$ représente le radical 2-octyl dodécyle, ladite composition (A) consistant en un mélange de composés représentés par les formules ($VII_1$), ($VII_2$), ($VII_3$), ($VII_4$) et ($VII_5$) :

$R_1\text{-}O\text{-}(G)_1\text{-}H$ ($VII_1$)

$R_1\text{-}O\text{-}(G)_2\text{-}H$ ($VII_2$)

$R_1\text{-}O\text{-}(G)_3\text{-}H$ ($VII_3$)

$R_1\text{-}O\text{-}(G)_4\text{-}H$ ($VII_4$)

$R_1\text{-}O\text{-}(G)_5\text{-}H$ ($VII_5$)

dans les proportions molaires respectives $a_1$, $a_2$, $a_3$, $a_4$ et $a_5$, telles que :

- La somme $a_1 + a_2 + a_3 + a_4 + a_5$ est égale à 1 et que
- La somme $a_1 + 2a_2 + 3a_3 + 4a_4 + 5a_5$ est égale à x ;

De 55% à 65% massique d'au moins un alcool gras de formule (VIII) :

$R'_1\text{-}OH$ (VIII),

dans laquelle $R'_1$ représente le radical 2-octyl dodécyle ;

De 10% à 30% massique d'au moins un polyhydroxystéarates de polyglycols représenté par la formule (XI) :

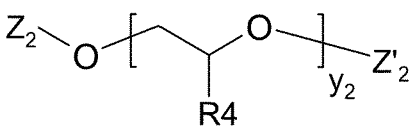

(XI)

dans laquelle yz représente un nombre entier supérieur ou égal à 2 et inférieur ou égal à 50, $R_4$ représente l'atome d'hydrogène, le radical méthyle ou le radical éthyle, $Z_2$ représente un radical de formule (XII) :

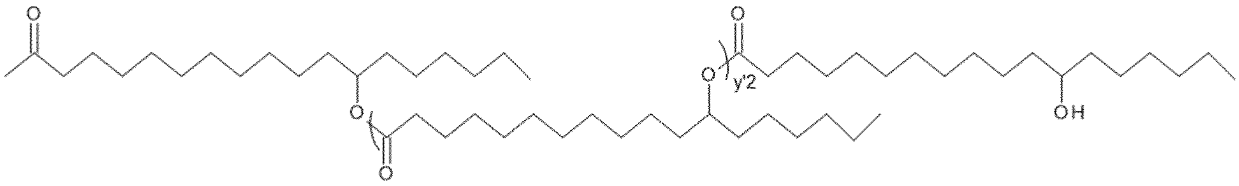

(XII),

dans laquelle $y'_2$ représente un nombre entier supérieur ou égal à 0 et inférieur ou égal à 10, plus particulièrement supérieur ou égal à 1 et inférieur ou égal à 10, $Z'_2$ représente un radical de formule (XII) telle que définie ci-dessus, avec $Z_2$' identique ou différent de $Z_2$, ou l'atome d'hydrogène.

Selon un autre aspect particulier, l'invention a pour objet une formulation (F) telle que définie précédemment caractérisée en ce que sa viscosité dynamique de ladite formulation (F), mesurée à une température de 25°C et à l'aide d'un viscosimètre Brookfield de type LVT à une vitesse de 6 tours/minute, est supérieure ou égale à 500 mPa.s et inférieure ou égale à 40 000 mPa.s.

La présente invention a également pour objet une composition à usage topique (F') se présentant sous la forme d'une émulsion huile-dans-eau et comprenant pour 100% de sa masse :

- de 50% à 90% massique, de préférence de 60 à 90% massique et encore plus préférentiellement de 70 à 90% massique d'une phase aqueuse (A1) cosmétiquement acceptable, ladite phase aqueuse (A1) comprenant pour 100% de sa propre masse de 0,5% à 10% massique du Lyzat (Ly) tel que défini précédemment.
- de 10% à 50% massique, de préférence de 10% à 40% massique et encore plus préférentiellement de 10% à 30% d'une phase grasse (G1) comprenant pour 100% de sa propre masse :

    - De 0,5% à 20% massique, de préférence de 1% à 15% massique d'au moins un agent tensioactif de type huile-dans-eau (S'1),
    - De 80% à 99,5% massique d'au moins une huile et/ou une cire.

Par « huile » présente dans la phase grasse ($G_1$) de la composition (F') se présentant sous la forme d'une émulsion de type huile-dans-eau telle que définie précédemment, on désigne au sens de la présente invention les substances chimiques ou les mélanges de substances chimiques insolubles dans l'eau, et se présentant sous un aspect liquide à une température de 25°C.

Par « cire » présente dans la phase grasse ($G_1$) de la composition (F') se présentant sous la forme d'une émulsion de type huile-dans-eau telle que définie précédemment, on désigne au sens de la présente invention les substances chimiques ou les mélanges de substances chimiques insolubles dans l'eau, et se présentant sous un aspect solide à une température de 45°C.

Par « agent tensioactif de type huile-dans-eau (S'1)» présent dans la phase grasse ($G_1$) de la composition (F') se présentant sous la forme d'une émulsion de type huile-dans-eau telle que définie précédemment, on désigne au sens de la présente invention, la substance chimique ou le mélange de substances chimiques qui permet de stabiliser les gouttelettes de ladite phase grasse ($G_1$) en dispersion dans la phase aqueuse continue ($A_1$).

Comme agent tensioactif de type huile-dans-eau (S'1) présent dans la phase grasse (G1) de l'émulsion (F') de type huile-dans-eau telle que définie précédemment, on peut citer par exemple:

- Des polysorbates résultant de la réaction d'éthoxylation entre un équivalent molaire d'esters de sorbitan et entre 5 et 20 équivalents molaires d'oxyde d'éthylène, et plus particulièrement entre un équivalent molaire de laurate de sorbitan, ou de palmitate de sorbitan, ou de stéarate de sorbitan, ou d'isostéarate de sorbitan, ou d'oléate de sorbitan, et entre 5 et 20 équivalents molaires d'oxyde d'éthylène, plus particulièrement 5 ou 10 ou 12 ou 15 ou 20 équivalents molaires d'oxyde d'éthylène;
- Les produits résultant de la réaction d'éthoxylation entre un équivalent molaire d'un acide gras, comme par exemple l'acide palmitique, l'acide myristique, l'acide laurique, l'acide stéarique, l'acide isostéarique, l'acide oléique, et entre 5 à 40 équivalents molaires d'oxyde d'éthylène;
- Les produits résultant de la réaction d'estérification entre un acide gras, comme par exemple l'acide palmitique, l'acide myristique, l'acide laurique, l'acide stéarique, l'acide isostéarique, l'acide oléique, l'acide arachidique, l'acide béhénique et entre 4 à 20 équivalents molaires, plus particulièrement entre 3 à 10 équivalents molaires de glycérol;

Par "phase grasse (G1)", on désigne au sens de la présente invention, un corps gras ou un mélange de corps gras insoluble dans l'eau et/ou dans les mélanges d'eau et de solvants polaires. Une telle « phase grasse » peut comprendre des huiles et/ou des cires telles que définies précédemment.

Parmi les éléments constitutifs de la phase grasse (G1) présente dans la formulation (F'), on peut citer les huiles constitutives de la phase grasse (A2) telles que décrites ci-dessus pour la préparation de la formulation (F), et les huiles de silicone comme les diméthylpolysiloxanes, les méthylphényl-polysiloxanes, les silicones modifiées par des amines, les silicones modifiées par des acides gras, les silicones modifiées par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Parmi les éléments constitutifs de la phase grasse (G1) présente dans la formulation (F'), on peut citer les cires constitutives de la phase grasse (A2) telles que décrites ci-dessus pour la préparation de la formulation (F).

Selon un aspect particulier, dans la formulation (F') objet de la présente invention, la phase grasse (G1) comprend en outre pour 100% de sa propre masse de 5% à 30% massique, plus particulièrement de 5% à 25% massique, et encore plus particulièrement de 10% à 25% massique d'au moins un agent de protection contre les rayonnements ultra-violets du soleil. Par agent de protection contre les rayonnements ultra-violets du soleil, on désigne notamment dans la définition de la composition (F') à usage topique se présentant sous la forme d'une émulsion de type huile-dans-eau et objet de la présente invention, les pigments, les filtres organiques solaires et les filtres inorganiques solaires.

Comme pigments utilisés comme agent de protection contre les rayonnements ultra-violets du soleil, il y a par exemple le dioxyde de titane, les oxydes de fer marrons, les oxydes de fer jaune, les oxydes de fer noir, ou les oxydes de fer rouges ou encore les pigments nacrés blancs ou colorés tels que les Mica-Titane.

Comme filtres organiques solaires utilisés comme agent de protection contre les rayonnements ultra-violets du soleil, il y a par exemple:

- Ceux de la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA;
- Ceux de la famille des dérivés de l'acide anthranilique comme l'homomenthyl-N-acétyl anthranilate;
- Ceux de la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle;
- Ceux de la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-$\alpha$-cyano-$\beta$-phényle, le cinnamate de 2-éthylhexyl-$\alpha$-cyano-$\beta$-phényle, le cinnamate de diparaméthoxy mono-2-éthylhexanoyl de glycéryle;
- Ceux de la famille des dérivés de la benzophénone comme la 2,4-dihydroxy benzophénone, la 2,2'-dihydroxy 4-méthoxy benzophénone, la 2,2',4,4'-tétrahydroxy benzophénone, la 2-hydroxy 4-méthoxy benzophénone, la 2-hydroxy 4-méthoxy 4'-méthyl benzophénone, la 2-hydroxy 4-méthoxy benzophénone-5-sulfonate, la 4-phényl benzophénone, le 2-éthylhexyl 4'-phényl benzophénone-2-carboxylate, la 2-hydroxy 4-n-octyloxy benzophénone, la 4-hydroxy 3-carboxy benzophénone ; le 3-(4'-méthylbenzylidène) d,l-camphre, le 3-(benzylidène)-d,l-camphre, le benzalkonium méthosulfate camphre ; l'acide urocanique, l'urocanate d'éthyle ;
- Ceux de la famille des dérivés de l'acide sulfonique comme l'acide 2-phényl benzimidazole-5-sulfonique et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy) 1,3,5-triazine, le 4,4-((6-(((1,1-diméthyléthyl) amino) carbonyl) phenyl) amino)-1,3,5-triazine-2,4-diyl diimino) bis-(2-éthylhexyl) ester de l'acide benzoïque, le 2-phényl-5-méthyl benzoxazole, le 2,2'-hydroxy-5-méthylphényl benzotriazole, le 2-(2'-hydroxy-5'-t-octylphényl) benzotriazole, le 2-(2'-hydroxy-5'-méthyphényl) benzotriazole; la dibenzazine; le dianisoylméthane, le 4-méthoxy-4"-t-butylbenzoylméthane ; la 5-(3,3-diméthyl-2-norbornylidène)-3-pentan-2-one ; le 2-(4-diethylamino 2-hydroxy benzoyl) benzoic acid hexyl ester, le 2,4-bis{[4-(2-ethylhexyloxy)-2-hydroxy] phenyl}-6-(4-methoxy phenyl) 1,3,5-triazine, le 2,4,6-tris[4-(2-ethylhexyloxycarbonyl) anilino]-1,3,5-triazine, le 2-ethylhexyl dimethoxybenzylidene dioxoimidazolidine propionate, la famille des dérivés du diphénylacrylate comme le 2-éthylhexyl-2-cyano-3,3-diphényl-2-propènoate, l'éthyl-2-cyano-3,3-diphényl-2-propènoate;
- Ceux de la famille des polysiloxanes comme le malonate de benzylidène siloxane. Comme filtres inorganiques solaires utilisés comme agent de protection contre les rayonnements ultra-violets du soleil, il y a par exemple: les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements

de surface et être éventuellement présentés sous formes de pré-dispersions aqueuses ou huileuses.

L'agent de protection contre les rayonnements ultra-violets du soleil sera de préférence choisi parmi les éléments du groupe constitué par dioxyde de titane, la 2,4-dihydroxy benzophénone, le 2-(4-diethylamino-2-hydroxybenzoyl) benzoic acid hexyl ester, le 2,4-bis{[4-(2-ethylhexyloxy)-2-hydroxy] phenyl}-6-(4-methoxyphenyl)-1,3,5-triazine, le 2,4,6-tris[4-(2-ethylhexyloxy carbonyl) anilino]-1,3,5-triazine et le 2-ethylhexyl dimethoxy benzylidene dioxoimidazolidine propionate.

L'expression "à usage topique" utilisée dans la définition de la composition (E1) se présentant sous la forme d'une émulsion de type huile-dans-eau telle que définie ci-dessus, signifie que ladite composition est formulée pour permettre son application sur la peau, les cheveux, le cuir chevelu ou les muqueuses, qu'il s'agisse d'une application directe dans le cas d'une composition cosmétique, dermocosmétique, dermopharmaceutique ou pharmaceutique ou d'une application indirecte par exemple dans le cas d'un produit d'hygiène corporelle sous forme de lingette en textile ou en papier ou de produits sanitaires destinés à être en contact avec la peau ou les muqueuses.

L'expression "cosmétiquement acceptable" utilisée dans la définition de la phase aqueuse ($A_1$) de la composition (E1) se présentant sous la forme d'une émulsion de type huile-dans-eau, signifie, selon la directive du Conseil de la Communauté Economique Européenne N°76/768/CEE du 27 juillet 1976 modifiée par la directive N°93/35/CEE du 14 juin 1993, toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Les compositions (F) et (F') pourront être administrables par voie topique pour améliorer l'hydratation de la peau et/ou du cuir chevelu et/ou des lèvres.

Les compositions (F) et (F') pourront en particulier être administrables par voie topique pour diminuer et/ou éliminer et/ou prévenir les gerçures et/ou les dartres et/ou les crevasses et/ou la dermatite atopique et/ou l'ichtyose et/ou des états de sécheresse de la peau ou des muqueuses, accompagnant des pathologies cutanées et/ou mucosales telles que l'eczéma.

**1) préparation d'un lysat selon l'invention (LY)**

Un lysat (LY) de cellules dédifférenciées de la plante *Helichrysum stoechas* est préparé par la mise en oeuvre du procédé précédemment décrit, et plus particulièrement par la mise en oeuvre :

- d'une étape a) de préparation de l'échantillon d'Helichrysum stoechas stérile, telle que décrite précédemment, en utilisant des feuilles d'Helichrysum stoechas découpées en fragments de quelques millimètres, puis stérilisée par passage successif :
- dans un bain d'éthanol à 70% et de Tween™ 80 (à hauteur 0.05%) pendant cinq minutes
- dans un bain d'eau de javel à 1% pendant cinq minutes.

Les feuilles sont ensuite rincées dans de l'eau distillée stérile à trois reprises successives.

- D'une étape b) de callogénèse, telle que décrite précédemment, avec un milieu de callogénèse dont la constitution est décrite dans le tableau 1 ci-dessus, avec une phase d'incubation d'une durée de trois semaines à une température de 20 à 25°C à la lumière.

- D'une étape c) de mise en suspension des cellules dédifférenciées, telle que décrite précédemment, avec un milieu de culture dont la composition est décrite dans le tableau 2 ci-dessus, pendant une durée de 14 à 30 jours, à une température de 20 à 25°C, à la lumière du jour, agitée par une agitation de type orbitale à une vitesse de 100 tours/minute.

- D'une étape d) de sélection d'une suspension fine telle que décrite précédemment, avec un prélèvement de 1/6 à 1/4 de culture obtenue à l'étape c), additionnée d'un volume de milieu de suspension tel que décrit dans le tableau 2 et en un volume ajusté au volume d'inoculum disponible dans le contenant.

La nouvelle suspension est mise en culture dans une enceinte climatique pour une durée de 14 jours, à une température de 20 à 25°C, à la lumière du jour, agitée par une agitation de type orbitale à une vitesse de 100 tours/minute.

- D'une étape e) de production de biomasse telle que décrite précédemment, avec un prélèvement de 1/6 à 1/4 du milieu obtenu à l'étape d) additionnée d'un volume de milieu de suspension tel que décrit dans le tableau 2, et en

un volume ajusté au volume d'inoculum disponible dans le contenant.

La nouvelle suspension est mise en culture dans une enceinte climatique pour une durée de 14 jours dans un bioréacteur à vagues, de capacité comprise entre 5 Litres et 1000 Litres, à une température de 20 à 25°C°C, à la lumière du jour, agitée par une agitation de type orbitale à une vitesse de 25 tours/minute.

- D'une étape f) d'homogénéisation à haute pression du milieu obtenu à l'issu de l'étape e), par passage dans un homogénéisateur à haute pression de marque GEA, à une pression de 500 bars sur une passe à une température inférieure à 40°C.
- On obtient ainsi un lysat (LY) comprenant pour 100% de sa masse :
- 90-98 % massique d'eau
- 10-2 % massique de matière sèche

D'une étape g) de stabilisation du milieu obtenu à l'étape f), en mélangeant 60% massique du milieu obtenu à l'étape f) avec 0,8% massique d'un agent épaississant commercialisé sous le nom de marque Solagum™AX, avec 37,9 % massique de glycérol, 0,3% de sorbate de potassium, de 1% de benzoate de sodium, mené à une valeur de 5.3 du pH

On obtient ainsi une composition $C_A$ comprenant pour 100% de sa masse :

- 60 % massique de lysat (LY)
- 37,9 % massique de glycérol
- 0,8 % massique de Solagum™ AX
- 0,3 % massique de sorbate de potassium
- 1 % massique de benzoate de sodium.

2) Mise en oeuvre expérimentale des effets biologiques d'un lysat de cellules dédifférenciées de la plante ***Helichrysum stoechas* selon l'invention**

Comme déjà mentionné le lysat tel que défini précédemment et obtenu par la mise en oeuvre d'un procédé comprenant les étapes a) à f) telles que décrites ci-dessus, permet de maintenir un bon niveau d'hydratation et de contribuer au confort des peaux sèches, réactives et/ou sensibles.

L'effet technique particulier du lysat (Ly) tel que défini précédemment et obtenu par la mise en oeuvre d'un procédé comprenant les étapes a) à f) telles que décrites ci-dessus, a été mis en évidence sur plusieurs modèles d'étude in vitro et in vivo.

Les expérimentations ont été réalisées en deux réplicats.

Les valeurs ont été exprimées en moyenne +/- sd [standard déviation = écart-type].

Pour chaque condition, les pourcentages de stimulation ou de protection ont été calculés comme suit :

% restauration = 100 x [moyenne(Composition CA) - moyenne (épidermes altérés)] / [moyenne(épidermes sains) - moyenne (épidermes altérés)].

A- Effet hydratant

Des tests ont ensuite été effectués afin de montrer l'effet hydratant du lysat (Ly) tel que défini précédemment.

Différentes analyses ont été réalisées afin d'étudier la restauration de la fonction barrière suite à une altération par la méthode « tape-stripping ».

Cette méthode consiste à appliquer et à presser un morceau de ruban adhésif (de marque « D-Squame ») sur la surface de la peau, puis à le retirer. Les premières applications enlèvent une couche cellulaire complète de cornéocytes. La quantité de cornéocytes diminue sur le ruban adhésif au fur et à mesure des applications.

Les rubans adhésifs « D-Squame » sont appliqués sur les explants en veillant à ce qu'ils soient toujours réalisés au même endroit et de la même manière, avec une pression constante du pouce. Le ruban adhésif est ensuite retiré d'un mouvement fluide, rapide et unidirectionnel afin d'obtenir un retrait homogène des cornéocytes.

Ce modèle permet d'évaluer l'impact de formulations cosmétiques sur la physiologie des épidermes dont la fonction barrière est altérée.

Selon le type d'évaluation de l'effet d'amélioration de l'hydratation de la peau et/ou du cuir chevelu et/ou des lèvres, une formulation ($F_1$) comprenant 1% de la composition $C_A$, est utilisée et aa constitution massique est la suivante.

[Table 3]

| Formulation ($F_1$) | |
|---|---|
| Ingrédients | Teneur en pourcentage massique |
| Eau | QSP 100 % |
| Glycérol | 1% |
| Montanov™ L(1) | 1 |
| Simulsol™ 165(2) | 1 |
| Triglycérides C8-C10<br>Caprilyc/Capric Triglycérides | 3 |
| DUB™ DNPG(3)<br>Neopentyl glycol diheptanoate | 7 |
| Sepimax™ ZEN (4)<br>Polyacrylate Crosspolymer-6 | 1 |
| Sepinov™ EMT 10 (5)<br>Hydroxyethyl Acrylate/Sodium Acryloyldimethyl Taurate Copolymer | 0.5 |
| Euxyl™ PE 9010 (6)<br>Phenoxylethanol - Ethylhexylglycerin | 1 |
| Sensiva™ PA40 (7)<br>3-Phenylpropan-1-ol -Octane-1,2-diol - Propane-1,3-diol | 0.5 |
| Composition $C_A$ | 1 |

(1) Montanov™L (nom INCI : C14-22 Alcohols & C12-20 Alkyl Glucoside) est une composition émulsionnante, destinée à stabiliser des émulsions, et utilisée pour la préparation de formulations cosmétiques.

(2) Simulsol™165 (nom INCI : PEG-100 Stéarate and Glyceryl Stéarate) est une composition émulsionnante, destinée à stabiliser des émulsions, notamment des émulsions huile-dans-eau, et utilisée pour la préparation de formulations cosmétiques.

(3) DUB™ DNPG (nom INCI : Neopentyl glycol diheptanoate" est un ester utilisé comme phase grasse et/ou agent émollient pour la préparation de formulations cosmétiques.

(4) Sepimax™ ZEN (nom INCI : Polyacrylate Crosspolymer-6) est un polyélectrolyte anionique réticulé utilisé comme agent épaississant et/ou émulsionnant et/ou stabilisant pour la préparation de formulations cosmétiques.

(5) Sepinov™ EMT 10 (nom INCI :Hydroxyethyl Acrylate/Sodium Acryloyldimethyl Taurate Copolymer" est un polyélectrolyte anionique réticulé utilisé comme agent épaississant et/ou émulsionnant et/ou stabilisant pour la préparation de formulations cosmétiques.

(6) Euxyl™ PE 9010 (nom INCI : Phenoxylethanol - Ethylhexylglycerin) est un agent conservateur utilisé pour la préparation de formulations cosmétiques.

(7) Sensiva™ PA40 (nom INCI : 3-Phenylpropan-1-ol -Octane-1,2-diol - Propane-1,3-diol) est un agent conservateur utilisé pour la préparation de formulations cosmétiques.

A.1 Mesure de la perte insensible à l'eau (ou PIE)

La mesure de la perte insensible à l'eau est mesurée par un appareil commercialisé sous le nom de marque TEWAMETER™300 par la société Courage & Khazaka.

La chambre de mesure cylindrique de l'appareil TEWAMETER™300 a été appliquée au-dessus de l'insert pour lequel un joint a permis d'assurer l'étanchéité entre la cellule de mesure et le tissu. La valeur de la vitesse d'évaporation

de l'eau (exprimée en g/m$^2$.h) a été déterminée automatiquement.

Les conditions environnementales pendant les expériences de mesure PIE (perte insensible en eau) ont été contrôlées, à une température stable de 20 ° C et avec un taux d'humidité relative de 50%.

Les explants présentant une fonction barrière altérée (par la méthode de "tape-stripping") caractérisés par une perte insensible en eau supérieure à 35 g/m$^2$.h, sont traités 1h après l'altération de la fonction barrière et tous les jours pendant cinq jours avec une formulation comprenant 1% de composition $C_A$. La perte insensible en eau est évaluée à l'issue de ces cinq jours de traitement.

Les expérimentations ont été réalisées en trois réplicats.

Les valeurs ont été exprimées en moyenne +/- sd [standard déviation = écart-type].

Pour chaque condition, les pourcentages d'effet ont été calculés comme suit :

$$\% \text{ effet} = [\text{moyenne (condition)}] / [\text{moyenne (épidermes altéré)}] \times 100 - 100$$

L'analyse statistique a été réalisée par un test de Student et un seuil de significativité fixé à 5%, en comparant les conditions deux à deux. Une différence entre l'efficacité de deux produits a été considérée :

- Significative si $p < 0{,}05$ ;
- Dite « à la limite de significativité » si $0{,}05 \leq p < 0{,}1$ ;
- Et non significative si $p > 0{,}1$.

Les résultats sont consignés dans le tableau 4 suivant :

[Table 4]

| | PIE (g/m$^2$.h) +/- SD | | % effet |
|---|---|---|---|
| | J0 | J5 | |
| Explants sains | - | 10,0 +/- 1,6 | - |
| Explants altérés | 41,8 +/- 3,9 | 36,6 +/- 4,2 | -13%LS |
| Explants altérés + Formulation ($F_1$) à 1% de la Composition $C_A$ | 52,0 +/- 3,5 | 37,1 +/- 4,5 | -29%*** |
| *Evolution de la perte insensible en eau* | | | |

*LS 0,1<p<0,05, *** p<0,001*

La diminution de la perte insensible en eau signifie une amélioration de la fonction barrière de la peau, autrement dit la peau est mieux hydratée.

Ainsi, le traitement des explants altérés par association à la Composition $C_A$ objet de la présente invention, permet de réduire la perte insensible en eau entre J0 et J5, témoignant ainsi d'un meilleur état d'hydratation de la peau.

A.2 Mesure des espaces intercellulaires

Par « espace intercellulaire », on désigne l'espace entre deux cellules voisines.Les explants présentant une fonction barrière altérée (par la méthode de "tape-stripping") caractérisés par une perte insensible en eau supérieure à 35 g/m$^2$.h, sont traités une heure après l'altération de la fonction barrière et tous les jours pendant cinq jours avec une formulation comprenant 1% de Composition $C_A$.

Les expérimentations ont été réalisées en trois réplicats.

Les espaces intercellulaires entre les kératinocytes, sont mesurée par la méthode consistant en une étude d'ultrastructure en Microscopie Electronique en Transmission (MET) à l'aide d'un microscope Hitachi HT7700.

Les tissus ont été fixés avec du glutaraldéhyde à 2% dans du tampon phosphate Sorensen 0,1 M (pH 7,4), colorés avec une solution aqueuse de 2% d'acétate d'uranyle puis déshydratés noyés dans de la résine époxy (Epon 812). Des coupes ultrafines (50 nm) ont été montées sur des grilles de cuivre.

Pour chaque condition, les espaces intercellulaires entre les kératinocytes de la couche basale de l'épiderme (couche la plus profonde de l'épiderme) ont été mesurés sur la base des clichés obtenus. Cette analyse n'a pas été réalisée sur explants sains.

Les expérimentations ont été réalisées en trois réplicats.

Les valeurs ont été exprimées en moyenne +/- sd [standard déviation = écart-type].

Pour chaque condition, les pourcentages d'effet ont été calculés comme suit :

$$\% \text{ effet} = [\text{moyenne (condition)}] / [\text{moyenne (épidermes altéré)}] \times 100 - 100$$

L'analyse statistique a été réalisée par un test de Student et un seuil de significativité fixé à 5%, en comparant les conditions deux à deux. Une différence entre l'efficacité de deux produits a été considérée :

- Significative si $p < 0{,}05$ ;
- Dite « à la limite de significativité » si $0{,}05 \leq p < 0{,}1$ ;
- Et non significative si $p > 0{,}1$.

[Table 5]

| | Espaces intercellulaires ($\mu$m) Moyenne +/- SD | % effet |
|---|---|---|
| explants altérés | 1,15 +/- 0,27 | - |
| Explants altérés + Formulation ($F_1$) à 1% de la Composition $C_A$ | 0,45 +/- 0,12 | -61%*** |
| mesure des espaces intercellulaires sur explants altérés<br>*** *p<0,001* | | |

La diminution des espaces intercellulaires entre les kératinocytes de la couche basale de l'épiderme d'une valeur de -61%, après une association entre les explants altérés et la Composition $C_A$ selon l'invention, traduit une meilleure cohésion ainsi qu'une meilleure organisation du tissu. Plus cohésif et mieux organisé, l'explant limite sa perte en eau ce qui entraîne la diminution de la perte insensible en eau et donc une amélioration de la fonction barrière de la peau et une meilleure hydratation de la peau.

A.3 Mesure de l'épaisseur du *stratum corneum*

Par « épaisseur du *stratum corneum* », on désigne l'épaisseur de la couche la plus superficielle de l'épiderme ou couche cornée.

Les explants présentant une fonction barrière altérée (par la méthode de "tape-stripping") caractérisés par une perte insensible en eau supérieure à 35 $g/m^2.h$, sont traités 1h après l'altération de la fonction barrière et tous les jours pendant cinq jours avec une formulation comprenant 1% de composition $C_A$.

Les expérimentations ont été réalisées en trois réplicats.

L'épaisseur du *Stratum corneum* est mesurée par la méthode consistant en une étude d'ultrastructure en Microscopie Electronique en Transmission (MET) à l'aide d'un microscope Hitachi HT7700.

Les tissus ont été fixés avec du glutaraldéhyde à 2% dans du tampon phosphate Sorensen 0,1 M (pH 7,4), colorés avec une solution aqueuse de 2% d'acétate d'uranyle puis déshydratés noyés dans de la résine époxy (Epon 812). Des coupes ultrafines (50 nm) ont été montées sur des grilles de cuivre.

Pour chaque condition, l'épaisseur du stratum corneum a été mesurée sur la base des clichés obtenus (quatre mesures par clichés). Cette analyse n'a pas été réalisée sur explants sains.

Les expérimentations ont été réalisées en trois réplicats.

Les valeurs ont été exprimées en moyenne +/- sd [standard déviation = écart-type].

Pour chaque condition, les pourcentages d'effet ont été calculés comme suit :

$$\% \text{ effet} = [\text{moyenne (condition)}] / [\text{moyenne (épidermes altéré)}] \times 100 - 100$$

L'analyse statistique a été réalisée par un test de Student et un seuil de significativité fixé à 5%, en comparant les conditions deux à deux. Une différence entre l'efficacité de deux produits a été considérée :

- Significative si $p < 0{,}05$ ;

- Dite « à la limite de significativité » si 0,05 ≤ p < 0,1 ;
- Et non significative si p > 0,1.

[Table 6]

| | Epaisseur du *stratum corneum* (μm) Moyenne +/- SD | % effet |
|---|---|---|
| explants altérés | 7,48 +/- 1,07 | - |
| explants altérés + Formulation ($F_1$) à 1% de la Composition $C_A$ | 13,26 +/- 2,33 | 77%*** |
| mesure de l'épaisseur du *stratum corneum* sur explants altérés<br>*** *p<0,001* | | |

Le *stratum corneum* est la couche cellulaire la plus superficielle de l'épiderme, le tissu le plus superficiel de la peau.

L'augmentation de l'épaisseur de cette couche de +77%, lorsqu'on associe la Composition $C_A$ selon l'invention à des explants altérés permet de limiter la perte en eau entraînant une meilleure hydratation de la peau et une amélioration de la restauration de la fonction barrière.

A.4 Mesure des facteurs biochimiques favorisant l'hydratation de la peau.

Par NMF (acronyme de « Natural Moisturizing Factor », on entend un facteur hydratant naturel comprenant un ensemble de substances hygroscopiques localisées à l'intérieur des cornéocytes, de l'épiderme.

Par « céramides », on entend des composés chimiques de la famille des céramides qui ont pour fonction de maintenir la cohésion des différents éléments de la peau. Les céramides forment une couche protectrice pour aider la peau à prévenir la déshydratation et à la protéger contre les agressions extérieures.

Les explants présentant une fonction barrière altérée (par la méthode de "tape-stripping") caractérisés par une perte insensible en eau supérieure à 35 $g/m^2.h$, sont traités 1h après l'altération de la fonction barrière et tous les jours pendant cinq jours avec une formulation comprenant 1% de la Composition $C_A$.

Les expérimentations ont été réalisées en deux réplicats.

Les valeurs ont été exprimées en moyenne +/- sd [standard déviation = écart-type].

Pour chaque condition, les pourcentages d'effet ont été calculés comme suit :

% effet = [moyenne (condition)] / [moyenne (épidermes altéré)] x 100 - 100

% restauration = 100 x [moyenne (Composition CA) - moyenne (épidermes altérés)] / [moyenne(épidermes sains) - moyenne (épidermes altérés)].

Les NMFs sont mesurés par la méthode de chromatographie LC-MS/MS utilisant un système HPLC Agilent 1100 (Agilent Technologies) couplé à un spectromètre de masse Micromass Quattro Micro API (Waters), équipé équipé d'une ionisation par électro-pulvérisation.Les valeurs obtenues à l'issue de la mesure des NMFs sont exprimées en microgrammes par milligrammes de protéines.

Les Céramides sont mesurés par la méthode de chromatographie LC-MS/MS utilisant un système consistant en une chromatographie liquide à haute pression ou HPLC UltiMate 3000 liquid (ThermoScientific) couplée à un spectromètre de masse MSQ Plus (ThermoScientific), équipé d'une ionisation chimique à pression atmosphérique (APCI).

Les valeurs obtenues à l'issue de la mesure des Céramides sont exprimées en unité arbitraire par milligramme de protéines.

[Table 7]

| | Céramides (UA/mg de prot) Moyenne +/- SD | % effet | restauration | NMFs (μg/mg de prot) Moyenne +/- SD | % effet | 96 restauration |
|---|---|---|---|---|---|---|
| explants sains | 48,56 +/- 3,56 | - | - | 54,46 +/- 3,85 | - | - |
| explants altérés | 28,13 +/- 2,81 | -42% vs explants sains | - | 29,24 +/- 3,43 | -46% vs explants sains | - |
| explants altérés + Formulation ($F_1$) à 1% de la Composition $C_A$ | 32,33 +/- 2,56 | +15% vs explants altérés | 24% | 36,49 +/- 2,73 | +25% vs explants altérés | 32% |
| mesure des Céramides et des NMFs sur des explants altérés non traités et traités par la composition $C_A$ selon l'invention *(pas de statistique - n=2)* | | | | | | |

La restauration de 24% de la quantité de céramides après un traitement des explants altérés par la composition $C_A$ selon l'invention, entraîne et traduit une amélioration de la restauration de la fonction barrière.

La restauration de 32% de la quantité de NMFs après des explants altérés par la composition $C_A$ selon l'invention entraîne et traduit une amélioration de la différenciation des kératinocytes et donc une amélioration de la restauration de la fonction barrière de la peau. La restauration de la fonction barrière de la peau permettant de prévenir la déshydratation de la peau et de la protéger contre les agressions extérieures.

A.5 Mesure du nombre de grains de kératohyaline

Les grains de kératohyaline sont des constituants de la couche granuleuse de la peau. L'augmentation du nombre de grains de kératohyaline (contenant de la pro-filaggrine) traduit une amélioration du processus de différenciation des kératinocytes.

Les explants présentant une fonction barrière altérée (par la méthode de "tape-stripping") caractérisés par une perte insensible en eau supérieure à 35 g/m$^2$.h, sont traités 1h après l'altération de la fonction barrière et tous les jours pendant cinq jours avec une formulation comprenant 1% de Composition $C_A$.

Les grains de kératohyaline sont mesurés par la méthode consistant en une étude d'ultrastructure en Microscopie Electronique en Transmission (MET) à l'aide d'un microscope Hitachi HT7700.

Les tissus ont été fixés avec du glutaraldéhyde à 2% dans du tampon phosphate Sorensen 0,1 M (pH 7,4), colorés avec une solution aqueuse de 2% d'acétate d'uranyle puis déshydratés noyés dans de la résine époxy (Epon 812). Des coupes ultrafines (50 nm) ont été montées sur des grilles de cuivre.

Pour chaque condition, une quantification des grains de kératohyaline a été réalisée sur la base des clichés obtenus. Cette analyse n'a pas été réalisée sur explants sains.

Les expérimentations ont été réalisées en trois réplicats.

Les valeurs ont été exprimées en moyenne +/- sd [standard déviation = écart-type].

Pour chaque condition, les pourcentages d'effet ont été calculés comme suit :

$$\% \text{ effet} = [\text{moyenne (condition)}] / [\text{moyenne (épidermes altéré)}] \times 100 - 100$$

L'analyse statistique a été réalisée par un test de Student et un seuil de significativité fixé à 5%, en comparant les conditions deux à deux. Une différence entre l'efficacité de deux produits a été considérée :

- Significative si p < 0,05 ;
- Dite « à la limite de significativité » si 0,05 ≤ p < 0,1 ;
- Et non significative si p > 0,1.

[Table 8]

| | Nombre de grains de kératohyaline Moyenne +/- SD | % effet |
|---|---|---|
| Explants altérés | 21,9 +/- 4,5 | - |
| + Explants altérés + Formulation ($F_1$) à 1% de la Composition $C_A$ | 32,6 +/- 5,6 | 49%*** |
| mesure du nombre de grains de kératohyaline sur les explants altérés et traités par la composition $C_A$ selon l'invention<br>*** *p<0,001* | | |

L'augmentation du nombre de grains de kératohyaline lorsque les explants altérés sont associés à la composition $C_A$ objet de la présente invention par rapport au nombre de grains de kératohyaline des explants altérés, traduit une amélioration du processus de différenciation des kératinocytes et par conséquent une amélioration du processus de différenciation des kératinocytes et donc une amélioration de la restauration de la fonction barrière de la peau.

A.6 Analyse des zones lacunaires de la peau humaine

Des analyses ont été réalisées afin d'étudier les zones lacunaires qui ont un impact sur l'effet hydratant.

Par zones lacunaires, on entend des zones hydrophiles, issues de la dégradation des cornéodesmosomes, présentes au niveau du *stratum corneum,* contenant de l'eau et de nombreuses protéines dont des enzymes impliquées dans le processing métabolique des lipides et des protéines.

L'analyse des zones lacunaires est réalisée par la méthode consistant en une étude d'ultrastructure en Microscopie Electronique en Transmission (MET) à l'aide d'un microscope Hitachi HT7700.

Les tissus ont été fixés avec du glutaraldéhyde à 2% dans du tampon phosphate Sorensen 0,1 M (pH 7,4), colorés avec une solution aqueuse de 2% d'acétate d'uranyle puis déshydratés noyés dans de la résine époxy (Epon 812). Des coupes ultrafines (50 nm) ont été montées sur des grilles de cuivre.

Pour chaque condition, une quantification des zones lacunaires a été réalisée sur la base des clichés obtenus. Cette analyse n'a pas été réalisée sur explants sains.

Les expérimentations ont été réalisées en trois réplicats.

Les valeurs ont été exprimées en moyenne +/- sd [standard déviation = écart-type].

Pour chaque condition, les pourcentages d'effet ont été calculés comme suit :

$$\% \text{ effet} = [\text{moyenne (condition)}] / [\text{moyenne (épidermes altéré)}] \times 100 - 100$$

L'analyse statistique a été réalisée par un test de Student et un seuil de significativité fixé à 5%, en comparant les conditions deux à deux. Une différence entre l'efficacité de deux produits a été considérée :

- Significative si p < 0,05 ;
- Dite « à la limite de significativité » si 0,05 ≤ p < 0,1 ;
- Et non significative si p > 0,1.

[Table 9]

| | Nombre de zones lacunaires Moyenne +/- SD | % effet |
|---|---|---|
| explants altérés | 2,7 +/- 0,6 | - |
| explants altérés + Formulation ($F_1$) à 1% de la Composition $C_A$ | 6,7 +/- 1,5 | 150%* |
| mesure du nombre de zones lacunaires sur les explants altérés et traités par la composition $C_A$ selon l'invention<br>* *p<0,05* | | |

Les explants altérés et traités avec la composition $C_A$ selon l'invention, présentent un plus grand nombre de

zones lacunaires que les explants altérés et non associés à la composition $C_A$, traduisant une teneur en eau plus élevée dans la peau anlaysée et donc une meilleure hydratation.

A.7 Analyse de la peau humaine par cornéométrie.

L'analyse par cornéométrie de la peau humaine a pour objet de déterminer l'état d'hydratation des couches supérieures de l'épiderme *(stratum corneum)* par la mesure des propriétés électriques de celles-ci.

Cette analyse par cornéométrie est réalisée par la méthode consistant en la mesure de capacitance du milieu diélectrique. Tout changement d'hydratation de la surface de la peau engendre une modification de ces constantes diélectriques, et par conséquent de la mesure de capacitance. Le cornéomètre mesure cette capacitance des couches les plus superficielles de l'épiderme, à l'aide d'une sonde appliquée verticalement sur la peau, avec une pression constante. Pendant la mesure, un champ électrique pénètre les couches superficielles de la peau et la constante diélectrique est mesurée. La mesure est obtenue 1 seconde après l'application. Elle est exprimée en unité arbitraire allant de 0 à 125 et désignent un indice d'hydratation (6-10 : peau très sèche - 10-50 : peau sèche - 50-125 : peau hydratée).

Le cornéomètre utilisé dans le cadre de cette étude était un Cormeometer® CM825 (Courage & Khazaka)

Ainsi, des femmes ont été recrutées pour évaluer l'effet hydratant de composition $C_A$ versus placebo et versus son équivalent en glycérine (0,364%). Elles ont appliqué les formulations (la formulation ($F_1$) décrite précédemment et la formulation ($F'_1$) ne se différenciant de ($F_1$) que par l'absence de la Composition $C_A$) deux fois par jour pendant 21 jours, avec mesure à J0, J7 et J21. L'hydratation a été évaluée par cornéométrie (mesure de la teneur en eau du *stratum corneum).*

Les résultats sont les suivants :

[Table 10]

| **Produit** | **Variation de l'indice d'hydratation (UA) entre J21-J0** |
|---|---|
| Placebo | +32,7% (p<0.0001) |
| Formulation ($F_1$) à 1% de la Composition $C_A$ | +46,7% (p<0.0001) |
| Glycerine 0.364% | +36,1% (p<0.0001) |
| | |
| **Produit** | **Variation de l'indice d'hydratation (UA) vs placebo à J21** |
| Formulation ($F_1$) à 1% de la Composition $C_A$ | 38,796 (p=0.0077) |
| | |
| **Produit** | **Variation de l'indice d'hydratation (UA) vs Glycerine 0.364%** à **J21** |
| Formulation ($F_1$) à 1% de la Composition $C_A$ | **31.65% (p=0.0397)** |

Cette étude montre les capacités hydratantes de la composition $C_A$ selon l'invention, après une durée de 21 jours.

**Conclusions générales**

En conclusion, les essais expérimentaux ont montré que la composition $C_A$ selon l'invention, comprenant le lysat (Ly) de cellules dédifférenciées de la plante Helichrysum stoechas, est capable d'augmenter et/ou de maintenir l'état d'hydratation de la peau humaine et/ou du cuir chevelu, plus particulièrement des peaux sèches et/ou sensibles et/ou réactives, en réduisant la perte insensible en eau, en diminuant les espaces intercellulaires entre les kératinocytes de la couche basale de l'épiderme, en augmentant l'épaisseur du *Stratum corneum,* stimulant la restauration de la barrière cutanée et en augmentant le nombre de zones lacunaires.

B) Formulations

Dans les formules suivantes, les pourcentages sont exprimés en poids de la formulation.

B.1 Fluide démaquillant visage

Formule

| | |
|---|---|
| Composition ($C_A$) | 10,00% |
| Méthyl paraben | 0,15% |
| Phenoxyethanol | 0,80% |
| SEPICALM™ S | 1,00% |
| Parfum/Fragrance | 0,10% |
| Eau qs. | 100,00% |

Mode opératoire :

Mélanger les différents ingrédients dans l'eau sous agitation magnétique dans l'ordre indiqué, et ajuster le pH aux alentours de 7.

B.2 Shampoing cheveux et corps pour enfants

Formule

A

| | |
|---|---|
| Composition ($C_A$) | 15,00% |
| PROTEOL™APL | 5,00% |
| SEPICIDE™HB | 0,50% |
| Parfum/Fragrance | 0,10% |

B

| | |
|---|---|
| Eau | 20,00% |
| CAPIGEL™98 | 3,50% |

C

| | |
|---|---|
| Eau Q.S. | 100,00% |
| SEPICIDE™CI | 0,30% |
| Colorant | Q.S |

| | |
|---|---|
| Soude | Q.S. pH = 7,2 |

Mode opératoire :

Mélanger la composition ($C_A$) avec le PROTEOL™APL, et le SEPICIDE™HB (Phase A). Diluer le CAPIGEL™98 dans une partie de l'eau et l'ajouter à la 30 phase A précédemment obtenue (Phase B). Ajouter le reste d'eau à la phase B, puis le SEPICIDE™CI et le colorant. Ajuster le pH du mélange à 7,2 environ avec de la soude.

B.3 Gel moussant doux

Formule

A

| | |
|---|---|
| Composition ($C_A$) | 8,50% |
| PROTEOL™ APL | 3,00% |
| EUXYL™ PE9010 | 1,00% |
| Parfum/Fragrance | 0,10% |

B

| | |
|---|---|
| Eau Q.S. | 100,00% |

Acide lactique Q.S. pH = 6,0

Mode opératoire :

Solubiliser le parfum et le conservateur EUXYL™ PE 9010 dans le mélange composé de la composition $C_A$ et du PROTEOL™ APL (phase A). Ajouter l'eau et régler le pH à environ 6,0 avec de l'acide lactique.

B.4 Shampoing à usage fréquent

Formule

A

| | |
|---|---|
| Composition ($C_A$) | 12,80% |
| PROTEOL™ OAT | 5,00% |
| EUXYL™ PE 9010 | 1,00% |
| Parfum/Fragrance | 0,30% |
| Eau Q.S. | 100,00% |

B

| | |
|---|---|
| MONTALINE™C40 | 8,50% |

| | |
|---|---|
| Acide lactique | Q.S. pH = 6,0 |

Mode opératoire : mélanger tous les ingrédients de la phase A et, après homogénéisation, ajouter la MONTALINE™C40 et ajuster le pH à environ 6,0 à l'aide de l'acide lactique

B.5 Lait de toilette pour bébé

Formule

A

| | |
|---|---|
| SIMULSOL™ 165 | 2,00% |

(suite)

| | |
|---|---|
| MONTANOV™202 | 1,00% |
| LANOL™99 | 3,00% |
| Dimethicone | 1,00% |
| Isohexadecane | 3,00% |

B

| | |
|---|---|
| Eau Q.S. | 100,00% |

C

| | |
|---|---|
| SEPIPLUS™400 | 0,30% |

D

| | |
|---|---|
| Composition ($C_A$) | 6,35% |

E

| | |
|---|---|
| SEPICIDE™HB | 0,30% |
| DMDM Hydantoin | 0,20% |
| Parfum/Fragrance | 0,10% |

Mode opératoire : Faire chauffer séparément les phases A et B constituées par mélange des différents constituants. Ajouter la phase C dans la phase grasse chaude et réaliser l'émulsion en versant la phase aqueuse ; homogénéiser quelques minutes sous forte agitation (par l'intermédiaire d'une turbine rotor/stator). Puis ajouter la phase D dans l'émulsion chaude et refroidir l'émulsion sous agitation modérée jusqu'à retour à température ambiante. Ajouter la phase E à 40°C.

B.6 Gel douche enfants Formule

A

| | |
|---|---|
| Eau | 56,06% |
| SEPIMAX™Zen | 3,00% |
| SEPIPLUS™S | 0,80% |

B

| | |
|---|---|
| PROTEOL™OAT | 20,80% |
| ORAMIX™NS 10 | 9,30% |
| AMONYL™265 BA | 5,10% |

C

| | |
|---|---|
| Composition ($C_A$) | 2,00% |
| Glycéryl Glucoside | 1,00% |
| Phenoxyéthanol & Ethylhexyl Glycérine | 1,00% |
| Parfum/Fragrance | 0,90% |
| Colorant | 0,04% |

Mode opératoire :

disperser le SEPIMAX™ZEN dans l'eau et agiter à l'aide d'un agitateur mécanique muni d'une défloculeuse, d'une contre-hélice et d'une pâle de type ancre, jusqu'à l'obtention d'un gel parfaitement lisse. Ajouter le SEPIPLUS™S puis agiter jusqu'à ce que le mélange soit homogène. Ajouter ensuite les ingrédients de la phase B, homogéniser et ajouter individuellement les additifs de la phase C. Ajuster le pH à 6.0 - 6.5.

B.7 BB Crème

Formule

A

| | |
|---|---|
| EASYNOV™ | 2,30% |
| LANOL™ 99 | 1,00% |
| SEPIMAT™ H10W | 1,00% |
| Ethylhexyl methoxycinnamate | 5,00% |

B

| | |
|---|---|
| Cyclométhicone | 6,00% |

| | |
|---|---|
| Triethoxycaprylsilane & Alumina-silane & Titanium Oxide 8,00% Iron Oxide red & Triethoxycaprylsilane | 0,24% |
| Iron Oxide yellow & Triethoxycaprylsilane | 0,66% |
| Iron Oxide black & Triethoxycaprylsilane | 0,09% |
| Parfum/Fragrance | 0,10% |

C

| | |
|---|---|
| Eau | qs 100% |
| SEPINOV™EMT10 | 1,20% |

D

| | |
|---|---|
| Composition ($C_A$) | 2,00% |
| SEPITONIC™M3 | 1,00% |
| Phenoxyéthanol & Ethylhexyl Glycérine | 1,00% |

Mode opératoire :

Préparer la phase B par mélange des différents ingrédients et homogénéiser à l'aide d'un mélangeur muni d'un système de rotor-stator à une vitesse de rotation de 4500 tours par minute, pendant une durée de 6 minutes. Préparer la phase C par addition du SEPINOV™EMT10 sur le mélange d'eau et de glycérol et homogénéiser à l'aide d'un mélangeur muni d'un système de rotor-stator à une vitesse de rotation de 4000 tours par minute pendant 4 minutes. Ajouter les phases A et B sur la phase C, et agiter le mélange résultant à l'aide d'un agitateur mécanique muni d'un pâle de type ancre, à une vitesse de 30 tours par minute pendant 2 minutes, puis à une vitesse de 50 tours par minute pendant 20 minutes. Ajouter un à un les composants de la phase et agiter à une vitesse de 50 tours par minute pendant 25 minutes.

B.8 Spray Solaire haute Protection SPH supérieur à 30

Formule

A

| | |
|---|---|
| MONTANOV™ L | 1,00% |
| MONTANOV™ 82 | 1,00% |
| C12-15 Alkylbenzoate | 17,00% |
| Dimethicone | 3,00% |
| Octocrylène | 6,00% |

| | |
|---|---|
| Ethylhexyl methoxycinnamate | 6,00% |
| Bis-ethylhexyloxyphenol Méthoxypenyl Triazine | 3,00% |
| Tocophérol | 0,05% |

B

| | |
|---|---|
| Eau | qs 100% |

C

| | |
|---|---|
| SIMULGEL™ INS 100 | 0,50% |
| Cyclodiméthicone | 5,00% |

D

| | |
|---|---|
| Composition ($C_A$) | 3,00% |
| Phenoxyéthanol & Ethylhexyl Glycérine | 1,00% |
| Parfum/Fragrance | 0,20% |

E

| | |
|---|---|
| Methylene Bis-Benzotriazolyl Tetraméthylbutylphénol | 10,00% |
| Acide citrique 25% | qs pH = 5 |

B.9 Bain hydratant d'imprégnation pour masques en papier

Formule

A

| | |
|---|---|
| Eau | qsp 100% |
| Glycérine | 2,00% |

B

| | |
|---|---|
| SEPIMAX™ Zen | 0,15% |

C

| | |
|---|---|
| Phénoxyéthanol et Ethylhexylglycérine | 1,00% |
| 1,2-Hexanediol | 1,00% |

D

| | |
|---|---|
| Composition $C_A$ | 1,00% |

SEPICALM™S : Mélange de N-cocoyl aminoacides, de sarcosine, d'aspartate de potassium et d'aspartate de magnésium tel que décrit dans WO 98/09611.

PROTEOL™APL : Mélange de sels de sodium de N-cocoyl aminoacides, obtenus par acylation des acides aminés caractéristiques du jus de pomme ;
SEPICIDE™HB : Mélange de phénoxyéthanol, de méthylparaben, d'éthylparaben, de propylparaben et de butylparaben, est un agent conservateur.
CAPIGEL™98 : Copolymère d'acrylates ;
SEPICIDE™CI : Imidazoline urée, est un agent conservateur ;
SEPICIDE™HB : Mélange de phénoxyéthanol, de méthylparaben, d'éthylparaben, de propylparaben, de butylparaben et d'isobutylparaben, est un agent conservateur ; EUXYL™PE9010 : Mélange de phénoxyéthanol et d'ethyl hexyl glycérine ;
PROTEOL™OAT : Mélange de N-lauryl aminoacides obtenus par hydrolyse totale de protéine d'avoine tel que décrit dans WO 94/26694 ;
MONTALINE™C40 : Sel de chlorure de Cocamidopropyl beta'inamide de Monoéthanolamine. SIMULSOL™165 : Mélange de stéarate de PEG-100 et de stéarate de glycérol ; SEPIPLUS™400 : Latex inverse auto-inversible de polyacrylates dans le polyisobutene et comportant du polysorbate 20, tel que décrit dans WO2005/040230 ;
SEPIMAX™Zen (nom INCI : Polyacrylate Crosspolymer-6) : Polymère épaississant se présentant sous la forme d'une poudre ;
SEPIPLUS™S (nom INCI: Hydroxyethyl Acrylate / Sodium Acryloyldimethyl Taurate Copolymer & Polyisobutene & PEG-7 Trimethylolpropane Cononut Ether): Latex inverse auto-inversible ;
ORAMIX™NS 10 (nom INCI : Decyl glucoside) est un agent moussant utilisé pour la préparation de formulations cosmétiques.
AMONYL™265 BA (nom INCI : Cocobétaïne) : Agent tensioactif amphotère moussant ; SEPINOV™EMT10 (nom INCI : Hydroxyethyl Acrylate / Sodium Acryloyldimethyl Taurate Copolymer) : Copolymère épaississant se présentant sous la forme d'une poudre ;
EASYNOV™ (nom INCI : Octyldodecanol and Octyldodecyl Xyloside and PEG-30 Dipolyhydroxystearate) : Agent émulsionnant à tendance lipophile ;
SEPIMAT™H10 FW (nom INCI :Methyl Méthacrylate Crosspolymer and Squalane) : polymère utilisé comme agent de texture ;
SEPITONIC™M3 (nom INCI : Magnésium Aspartate and Zinc Gluconate and Copper Gluconate) : Mélange utilisé comme agent antiradicalaire et énergisant pour les cellules ; MONTANOV™L (nom INCI : C14-22 Alcohols and C12-20 Alkylglucoside) : Agent émulsionnant ;
MONTANOV™82 (nom INCI : Cetearyl Alcohol and Coco-glucoside) : Agent émulsionnant ; SIMULGEL™INS100 (nom INCI :Hydroxyethyl Acrylate/Sodium Acryloydimethyl Taurate Copolymer and isohexadecane and Polysorbate 60) : Agent épaississant polymèrique ;

**Revendications**

1. Lysat (Ly) de cellules dédifférenciées de la plante *Helichrysum stoechas* sous une forme adaptée à une administration par voie topique pour améliorer l'hydratation de la peau et/ou du cuir chevelu et/ou des lèvres.

2. Lysat (Ly) selon la revendication 1, **caractérisé en ce qu'**il est issu de l'homogénéisation haute pression de la culture de cellules dédifférenciées de la plante *Helichrysum stoechas.*

3. Composition à usage topique (C1) se présentant sous la forme d'un gel et comprenant pour 100% de sa masse :

   - De 95% à 99,5% massique du Lysat (Ly) tel que défini à la revendication 1 ou 2

- De 0,5% à 5% massique d'au moins un agent épaississant et/ou gélifiant.

**4.** Composition (C1) selon la revendication 3, **caractérisée en ce que** les agents gélifiants et/ou épaississants sont choisis parmi les polysaccharides, la cellulose et les dérivés de la cellulose, les amidons, et les polymères de type polyélectrolytes, linéaires ou branchés ou réticulés.

**5.** Composition (C1) selon la revendication 3 ou 4 sous une forme adaptée à une administration par voie topique pour améliorer l'hydratation de la peau et/ou du cuir chevelu et/ou des lèvres.

**6.** Composition à usage topique (C'1) se présentant sous la forme d'un gel et comprenant pour 100% de sa masse :

- De 50% à 80% massique du Lysat (Ly) tel que défini à la revendication 1 ou 2,
- De 0,1% à 5% massique d'au moins un agent épaississant et/ou gélifiant, et
- De 15% à 49,9% massique d'au moins un solvant.

**7.** Composition (C'1) selon la revendication 6, **caractérisé en ce que** le solvant est choisi parmi les éléments du groupe constitué par :

- les composés de formule (la) :

HO-[CH2-CH(OH)-CH2-O]n-H (la)

dans laquelle n représente un nombre entier supérieur ou égal à un et inférieur ou égal à quinze, plus particulièrement supérieur ou égal à un et inférieur ou égal à 10, plus particulièrement supérieur ou égal à un et inférieur ou égal à 6, plus particulièrement supérieur ou égal à un et inférieur ou égal à 4, plus particulièrement égal à 1 ou 2 ou 3 ou 4 ;
- les composés de formule (Ib) :

Ra1-C(Rb1)(OH)-C(OH)(Rc1)(Rd1) (Ib),

dans laquelle chacun des radicaux Ra1, Rb1, Rc1 et Rd1 représentent indépendamment les uns des autres, un atome d'hydrogène ou radical aliphatique saturé comportant de un à cinq atomes de carbone, soit par la formule (Ib1) :

Ra1-C(Rb1)(OH)-[C(Re1)(Rf1)]t-C(OH)(Rc1)(Rd1) (Ib1),

dans laquelle t est égal à un, deux ou trois, chacun des radicaux Ra1, Rb1, Rc1, Rd1, Re1 et Rf1 représentent indépendamment les uns des autres, un atome d'hydrogène ou radical aliphatique saturé comportant de un à cinq atomes de carbone, étant entendu que l'un au moins des radicaux Ra1 ou Rb1 et/ou l'un au moins des radicaux Rc1 ou Rd1 ne représentent pas un atome d'hydrogène.

**8.** Composition (C'1) selon la revendication 6 ou 7, **caractérisée en ce que** le solvant est le glycérol.

**9.** Composition (C'1) selon l'une des revendications 6 à 8 sous une forme adaptée à une administration par voie topique pour améliorer l'hydratation de la peau et/ou du cuir chevelu et/ou des lèvres.

**10.** Composition à usage topique (C2) se présentant sous la forme d'une émulsion de type eau-dans-huile ou d'une émulsion de type huile-dans-eau, comprenant le lysat (Ly) tel que défini à la revendication 1 ou 2.

**11.** Composition à usage topique (F) se présentant sous la forme d'une émulsion eau-dans-huile et comprenant pour 100% de sa masse :

- de 60% à 90% massique de la composition à usage topique (C1) ou (C'1) telle que définie à l'une des revendications 3 à 9,
- de 10 % à 40 % massique d'une phase grasse ($A_2$) comprenant i) au moins une huile, et optionnellement au moins une cire et ii) un système émulsionnant comprenant au moins un agent tensioactif émulsionnant ($S_1$).

**12.** Composition (F) selon la revendication 11, **caractérisée en ce que** l'agent tensioactif émulsionnant ($S_1$) est choisi

parmi les éléments du groupe constitué par les compositions d'alkylpolyglycosides, les compositions d'alkylpolyglycosides et d'alcools gras, les esters de polyglycérols, les esters de polyglycérols alcoxylés, les polyhydroxystéarates de polyglycols, les polyhydroxystéarates de polyglycérols, les polyhydroxystéarates de polyglycérols alcoxylés.

**13.** Composition à usage topique (F') se présentant sous la forme d'une émulsion huile-dans-eau et comprenant pour 100% de sa masse :

- de 50% à 90% massique d'une phase aqueuse (A1) cosmétiquement acceptable, ladite phase aqueuse (A1) comprenant pour 100% de sa propre masse de 0,5% à 10% massique du Lysat (Ly) tel que défini à l'une des revendications 1 ou 2.
- de 10% à 50% massique d'une phase grasse (G1) comprenant pour 100% de sa propre masse :
  - De 0,5% à 20% massique d'au moins un agent tensioactif de type huile-dans-eau (S'1),
  - De 80% à 99,5% massique d'au moins une huile et/ou une cire.

**14.** Composition (C2), (F) ou (F') selon l'une des revendications 10 à 13 sous une forme adaptée à une administration par voie topique pour améliorer l'hydratation de la peau et/ou du cuir chevelu et/ou des lèvres.

**15.** Composition (C2), (F) ou (F') selon l'une des revendications 10 à 13 sous une forme adaptée à une administration par voie topique pour diminuer et/ou éliminer et/ou prévenir les gerçures et/ou les dartres et/ou les crevasses et/ou la dermatite atopique et/ou l'ichtyose et/ou des états de sécheresse de la peau ou des muqueuses, accompagnant des pathologies cutanées et/ou mucosales telles que l'eczéma.

## Patentansprüche

**1.** Lysat (Ly) von dedifferenzierten Zellen der Pflanze *Helichrysum stoechas* in einer für die topische Verabreichung geeigneten Form zur Verbesserung der Feuchtigkeit der Haut und/oder der Kopfhaut und/oder der Lippen.

**2.** Lysat (Ly) nach Anspruch 1, **dadurch gekennzeichnet, dass** es aus der Hochdruckhomogenisierung der Kultur von dedifferenzierten Zellen der Pflanze *Helichrysum stoechas* stammt.

**3.** Zusammensetzung zur topischen Verwendung (C1), die in Form eines Gels vorliegt und auf 100 Gew.-% ihrer Masse

- 95 bis 99,5 Massen-% des Lysats (Ly) gemäß Anspruch 1 oder 2 und
- 0,5 bis 5 Massen-% mindestens eines Verdickungsmittels und/oder Gelierungsmittels umfasst.

**4.** Zusammensetzung (C1) nach Anspruch 3, **dadurch gekennzeichnet, dass** die Verdickungsmittel und/oder Gelierungsmittel aus Polysacchariden, Cellulose und Cellulosederivaten, Stärken und linearen oder verzweigten oder vernetzten Polymeren vom Polyelektrolyt-Typ ausgewählt sind.

**5.** Zusammensetzung (C1) nach Anspruch 3 oder 4 in einer für die topische Verabreichung geeigneten Form zur Verbesserung der Feuchtigkeit der Haut und/oder der Kopfhaut und/oder der Lippen.

**6.** Zusammensetzung zur topischen Verwendung (C'1), die in Form eines Gels vorliegt und auf 100 Gew.-% ihrer Masse

- 50 bis 80 Massen-% des Lysats (Ly) gemäß Anspruch 1 oder 2,
- 0,1 bis 5 Massen-% mindestens eines Verdickungsmittels und/oder Gelierungsmittels und
- 15 bis 49,9 Massen-% mindestens eines Lösungsmittels umfasst.

**7.** Zusammensetzung (C'1) nach Anspruch 6, **dadurch gekennzeichnet, dass** das Lösungsmittel aus den Elementen der Gruppe bestehend aus

- Verbindungen der Formel (Ia): $HO-[CH_2-CH(OH)-CH_2-O]_n-H$ (Ia), in der n für eine ganze Zahl größer oder gleich eins und kleiner oder gleich fünfzehn, spezieller größer oder gleich eins und kleiner oder gleich 10, spezieller größer oder gleich eins und kleiner oder gleich 6, spezieller größer oder gleich eins und kleiner oder gleich 4, spezieller gleich 1 oder 2 oder 3 oder 4, steht;
- Verbindung der Formel (Ib): Ra1-C(Rb1) (OH)-C(OH) (Rc1) (Rd1) (Ib), in der jeder der Reste Ra1, Rb1, Rc1

und Rd1 unabhängig voneinander für ein Wasserstoffatom oder einen gesättigten aliphatischen Rest mit eins bis fünf Kohlenstoffatomen steht, oder der Formel (Ib1):

Ra1-C(Rb1) (OH)-[C(Re1) (Rf1)]t-C(OH) (Rc1) (Rd1) (Ib1), in der t gleich eins, zwei oder drei ist und jeder der Reste Ra1, Rb1, Rc1, Rd1, Re1 und Rf1 unabhängig voneinander für ein Wasserstoffatom oder einen gesättigten aliphatischen Rest mit eins bis fünf Kohlenstoffatomen steht, mit der Maßgabe, dass mindestens einer der Reste Ra1 und Rb1 und/oder mindestens einer der Reste Rc1 und Rd1 nicht für ein Wasserstoffatom steht; ausgewählt ist.

**8.** Zusammensetzung (C'1) nach Anspruch 6 oder 7, **dadurch gekennzeichnet, dass** es sich bei dem Lösungsmittel um Glycerin handelt.

**9.** Zusammensetzung (C'1) nach einem der Ansprüche 6 bis 8 in einer für die topische Verabreichung geeigneten Form zur Verbesserung der Feuchtigkeit der Haut und/oder der Kopfhaut und/oder der Lippen.

**10.** Zusammensetzung zur topischen Verwendung (C2), die in Form einer Emulsion vom Wasser-in-Öl-Typ oder einer Emulsion vom Öl-in-Wasser-Typ vorliegt, umfassend das Lysat (Ly) gemäß Anspruch 1 oder 2.

**11.** Zusammensetzung zur topischen Verwendung (F), die in Form einer Wasser-in-Öl-Emulsion vorliegt und auf 100 Gew.-% ihrer Masse

- 60 bis 90 Massen-% der Zusammensetzung zur topischen Verwendung (C1) oder (C'1) gemäß einem der Ansprüche 3 bis 9 und
- 10 bis 40 Massen-% einer Fettphase ($A_2$), umfassend i) mindestens ein Öl und gegebenenfalls mindestens ein Wachs und ii) ein Emulgiersystem, das mindestens ein emulgierendes Tensid ($S_1$) umfasst,

umfasst.

**12.** Zusammensetzung (F) nach Anspruch 11, **dadurch gekennzeichnet, dass** das emulgierende Tensid ($S_1$) aus Elementen der Gruppe bestehend aus Zusammensetzungen von Alkylpolyglykosiden, Zusammensetzungen von Alkylpolyglykosiden und Fettalkoholen, Polyglycerinestern, alkoxylierten Polyglycerinestern, Polyglykolpolyhydroxystearaten, Polyglycerinpolyhydroxystearaten und alkoxylierten Polyglycerinpolyhydroxystearaten ausgewählt ist.

**13.** Zusammensetzung zur topischen Verwendung (F'), die in Form einer Wasser-in-Öl-Emulsion vorliegt und auf 100 Gew.-% ihrer Masse

- 50 bis 90 Massen-% einer kosmetisch unbedenklichen wässrigen Phase (A1), wobei die wässrige Phase (A1) auf 100 % ihrer eigenen Masse, 0,5 bis 10 Massen-% Lysat (Ly) gemäß Anspruch 1 oder 2 umfasst,
- 10 bis 50 Massen-% einer Fettphase (G1), umfassend auf 100 % ihrer eigenen Masse
  - • 0,5 bis 20 Massen-% mindestens eines Tensids vom Öl-in-Wasser-Typ (S'1),
  - • 80 bis 99,5 Massen-% mindestens eines Öls und/oder eines Wachses,

umfasst.

**14.** Zusammensetzung (C2), (F) oder (F') nach einem der Ansprüche 10 bis 13 in einer für die topische Verabreichung geeigneten Form zur Verbesserung der Feuchtigkeit der Haut und/oder der Kopfhaut und/oder der Lippen.

**15.** Zusammensetzung (C2), (F) oder (F') nach einem der Ansprüche 10 bis 13 in einer für die topische Verabreichung geeigneten Form zur Verringerung und/oder Beseitigung und/oder Verhinderung von Schrunden und/oder Flechten und/oder Rissen und/oder atopischer Dermatitis und/oder Ichthyose und/oder Trockenheit der Haut oder der Schleimhäute in Verbindung mit Haut- und/oder Schleimhautpathologien wie Ekzem.

## Claims

**1.** Lyzate (Ly) of dedifferentiated cells of the plant *Helichrysum stoechas* in a form that is suitable for topical administration, for improving the moisturization of the skin and/or the scalp and/or the lips.

2. Lyzate (Ly) according to Claim 1, **characterized in that** it results from the high-pressure homogenization of the culture of dedifferentiated cells of the plant *Helichrysum stoechas*.

3. Composition (C1) for topical use which is in the form of a gel and which comprises, per 100% of its mass:

- from 95% to 99.5% by mass of the lyzate (Ly) as defined in Claim 1 or 2,
- from 0.5% to 5% by mass of at least one thickener and/or gelling agent.

4. Composition (C1) according to Claim 3, **characterized in that** the gelling agents and/or thickeners are chosen from polysaccharides, cellulose and cellulose derivatives, starches and linear or branched or crosslinked polymers of polyelectrolyte type.

5. Composition (C1) according to Claim 3 or 4, in a form which is suitable for topical administration, for improving the moisturization of the skin and/or the scalp and/or the lips.

6. Composition (C'1) for topical use which is in the form of a gel and which comprises, per 100% of its mass:

- from 50% to 80% by mass of the lyzate (Ly) as defined in Claim 1 or 2,
- from 0.1% to 5% by mass of at least one thickener and/or gelling agent, and
- from 15% to 49.9% by mass of at least one solvent.

7. Composition (C'1) according to Claim 6, **characterized in that** the solvent is chosen from the elements of the group consisting of:

- the compounds of formula (Ia):

$$HO\text{-}[CH_2\text{-}CH(OH)\text{-}CH_2\text{-}O]_n\text{-}H \qquad (Ia)$$

in which n represents an integer greater than or equal to 1 and less than or equal to 15, more particularly greater than or equal to 1 and less than or equal to 10, more particularly greater than or equal to 1 and less than or equal to 6, more particularly greater than or equal to 1 and less than or equal to 4, more particularly equal to 1 or 2 or 3 or 4;
- the compounds of formula (Ib):

Ra1-C(Rb1) (OH)-C(OH) (Rc1) (Rd1) ( Ib),

in which each of the radicals Ra1, Rb1, Rc1 and Rd1 represent, independently of each other, a hydrogen atom or a saturated aliphatic radical including from 1 to 5 carbon atoms, or by the formula (Ib1):

$$Ra1\text{-}C(Rb1)\,(OH)\text{-}[C(Re1)\,(Rf1)]_t\text{-}C(OH)\,(Rc1)\,(Rd1)\ (Ib1),$$

in which t is equal to 1, 2 or 3 and each of the radicals Ra1, Rb1, Rc1, Rd1, Re1 and Rf1 represent, independently of each other, a hydrogen atom or a saturated aliphatic radical including from 1 to 5 carbon atoms, it being understood that at least one of the radicals Ra1 or Rb1 and/or at least one of the radicals Rc1 or Rd1 does not represent a hydrogen atom.

8. Composition (C'1) according to Claim 6 or 7, **characterized in that** the solvent is glycerol.

9. Composition (C'1) according to one of Claims 6 to 8, which is in a form that is suitable for topical administration, for improving moisturization of the skin and/or the scalp and/or the lips.

10. Composition (C2) for topical use which is in the form of an emulsion of water-in-oil type or of an emulsion of oil-in-water type, comprising the lyzate (Ly) as defined in Claim 1 or 2.

11. Composition (F) for topical use which is in the form of a water-in-oil emulsion and which comprises, per 100% of its mass:

- from 60% to 90% by mass of composition (C1) or (C'1) for topical use as defined in one of Claims 3 to 9,
- from 10% to 40% by mass of a fatty phase ($A_2$) comprising i) at least one oil, and optionally at least one wax, and ii) an emulsifying system comprising at least one emulsifying surfactant ($S_1$).

**12.** Composition (F) according to Claim 11, **characterized in that** the emulsifying surfactant ($S_1$) is chosen from the elements of the group consisting of alkylpolyglycoside compositions, compositions of alkylpolyglycosides and of fatty alcohols, polyglycerol esters, alkoxylated polyglycerol esters, polyglycol polyhydroxystearates, polyglycerol polyhydroxystearates and alkoxylated polyglycerol polyhydroxystearates.

**13.** Composition (F') for topical use which is in the form of an oil-in-water emulsion and which comprises, per 100% of its mass:

- from 50% to 90% by mass of a cosmetically acceptable aqueous phase (A1), said aqueous phase (A1) comprising, per 100% of its own mass, from 0.5% to 10% by mass of the lyzate (Ly) as defined in either of Claims 1 and 2,
- from 10% to 50% by mass of a fatty phase (G1) comprising, per 100% of its own mass:
    - from 0.5% to 20% by mass of at least one surfactant of oil-in-water type (S'1),
    - from 80% to 99.5% by mass of at least one oil and/or one wax.

**14.** Composition (C2), (F) or (F') according to one of Claims 10 to 13, which is in a form that is suitable for topical administration, for improving the moisturization of the skin and/or the scalp and/or the lips.

**15.** Composition (C2), (F) or (F') according to one of Claims 10 to 13, which is in a form that is suitable for topical administration, for reducing and/or eliminating and/or preventing chapping and/or dry patches and/or cracking and/or atopic dermatitis and/or ichthyosis and/or dryness of the skin or mucous membranes, accompanying cutaneous and/or mucosal pathologies such as eczema.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0234793 A2
- WO 9809611 A
- WO 9426694 A
- WO 2005040230 A


### Littérature non-brevet citée dans la description


- **DANIEL VOET ; JUDITH G. VOET.** Biochemistry. John Wyley & Sons, 1990, 250